# EUROPEAN PATENT SPECIFICATION

(11) **EP 0 955 294 B1**
(45) Date of publication and mention of the grant of the patent: **24.09.2003**
(21) Application number: 99115614.2
(22) Date of filing: 07.06.1990
(51) Int. Cl.: C07D 233/58, C07D 233/70, C07D 233/82, C07D 401/12, C07D 403/12, C07D 405/12, C07D 409/12, A61K 31/415, C07D 233/54, C07D 401/06, C07D 405/06, C07D 409/14

(54) **Imidazolyl-alkenoic acid**
Imidazoalkensäure
Acides imidazoalcénoiques

(30) Priority: 14.06.1989 US 366079; 06.04.1990 US 506412
(43) Date of publication of application: 10.11.1999
(62) Divisional of application: 90306204.0
(73) Proprietor: SMITHKLINE BEECHAM CORPORATION, Philadelphia Pennsylvania 19101 (US)
(72) Inventor: Finkelstein, Joseph Alan, Philadelphia, PA 19151 (US); Keenan, Richard McCulloch, Malvern, pA 19355 (US); Weinstock, Joseph, Phoenixville, PA 19460 (US)
(74) Representative: Blakey, Alison Jane

(56) References cited:
- EP-A- 0 028 833
- EP-A- 0 028 834
- EP-A- 0 103 647
- EP-A- 0 253 310
- EP-A- 0 294 973
- EP-A- 0 324 377

## Description

The present invention relates to new imidazolyl-alkenoic acids which are angiotensin II receptor antagonists and are useful in regulating hypertension induced or exacerbated by angiotensin II, and in the treatment of congestive heart failure, renal failure, and glaucoma. This invention also relates to pharmaceutical compositions containing these compounds and methods for using these compounds as antagonists of angiotensin II, as antihypertensive agents and as agents for treating congestive heart failure, renal failure, and glaucoma.

### BACKGROUND OF THE INVENTION

The class of peptide pressor hormone known as angiotensin is responsible for a vasopressor action that is implicated in the etiology of hypertension in man. Inappropriate activity of the renin-angiotensin systems appears to be a key element in essential hypertension, congestive heart failure and in some forms of renal disease. In addition to a direct action on arteries and arterioles, angiotensin II (AII), being one of the most potent endogenous vasoconstrictors known, exerts stimulation on the release of aldosterone from the adrenal cortex. Therefore, the renin-angiotensin system, by virtue of its participation in the control of renal sodium handling, plays an important role in cardiovascular hemeostasis.

Interruption of the renin-angiotensin system with converting enzyme inhibitors, such as captopril, has proved to be clinically useful in the treatment of hypertension and congestive heart failure (Abrams, W.B., et al., (1984), Federation Proc., 43, 1314). The most direct approach towards inhibition of the renin-angiotensin system would block the action of All at the receptor. Compelling evidence suggests that All also contributes to renal vasoconstriction and sodium retention that is characteristic of a number of disorders such as heart failure, cirrhosis and complications of pregnancy (Hollenberg, N.K., (1984), J. Cardiovas. Pharmacol., 6, S176). In addition, recent animal studies suggest that inhibition of the renin-angiotensin system may be beneficial in halting or slowing the progression of chronic renal failure (Anderson, S., et al., (1985), J. Clin. Invest., 76, 612). Also, a recent patent application (South African Patent Application No. 87/01,653) claims that All antagonists are useful as agents for reducing and controlling elevated intraocular pressure, especially glaucoma, in mammals.

The compounds of this invention inhibit, block and antagonize the action of the hormone AII, and are therefore useful in regulating and moderating angiotensin induced hypertension, congestive heart failure, renal failure and other disorders attributed to the actions of AII. When compounds of this invention are administered to mammals, the elevated blood pressure due to All is reduced and other manifestations based on All intercession are minimized and controlled. Compounds of this invention are also expected to exhibit diuretic activity.

Recognition of the importance of blocking and inhibiting the actions of AH has stimulated other efforts to synthesize antagonists of AII. The following references have disclosed imidazole derivatives which are described as having All blocking activity and useful as hypotensive agents.

Furukawa et al., U.S. Patent 4,340,598 discloses imidazol-5-yl acetic acids and imidazol-5-yl-propahoic acids. Specifically, the discloser includes 1-benzyl-2-n-butyl-5-chloroimidazole-4-acetic acid and 1-benzyl-2-phenyl-5-chloro-imidazole-4-propanoic acid.

Furukawa, et al., U.S. Patent 4,355,040 discloses substituted imidazole-5-acetic acid derivatives. A compound specifically disclosed is 1-(2-chlorobenzyl)-2-n-butyl-4-chloroimidazole-5-acetic acid.

Carini et al. in EP 253,310 disclose certain imidazolylpropenoic acids. Two intermediates described in this patent are ethyl 3-[1-(4-nitrobenzyl)-2-butyl-4-chloroimidazol--5-yl]propenoate and ethyl 3-[2-butyl-4-chloro-1-(4-aminobenzyl)imidazol-5-yl]propenoate.

Also, Wareing, in PCT/EP 86/00297, discloses as intermediates certain imidazolylpropenoate compounds. On page 62, Formula (CX) is ethyl 3-[1-(4-fluorophenyl)-4-isopropyl-2-phenyl-1H-imidazol-5-yl]-2-propenoate.

### DESCRIPTION OF THE INVENTION

The present invention relates to blockers of angiotensin II receptors represented by the following Formula (I): 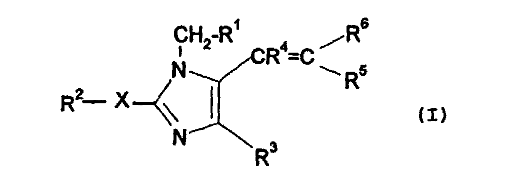 in which:
R¹ is phenyl unsubstituted or substituted by one to three substituents selected from chloro, fluoro, trifluoromethyl, nitro, methyl, methoxy, hydroxy, sulfamido, carboxy, carboC₁-C₄alkoxy, carbamoyl, cyano, or tetrazol-5-yl;
X is a single bond;
R² is C₂-C₈alkyl;
R³ is hydrogen;
R⁴ is hydrogen;
R⁵ is thienylmethyl optionally substituted by methyl or methoxy; and
R⁶ is COOH; or a pharmaceutically acceptable salt thereof.

The E isomers (trans stereochemistry of the R⁶ group and imidazole group) are generally more active and thus, are preferred over the Z isomers (cis).

As used herein, the terms alkyl, alkenyl, alkoxy and alkynyl mean carbon chains which are branched or unbranched with the length of the chain determined by the descriptor preceding the term.

Particular compounds of the invention include, but are not limited to, the following:
(E)-3-[2-n-butyl-1-{(2-chlorophenyl)methyl}-1H-imidazol-5-yl]-2-(2-thienyl)methyl-2-propenoic acid,
(E)-3-[2-n-butyl-1-{(2-chlorophenyl)methyl}-1H-imidazol-5-yl]-2-(3-thienyl)methyl-2-propenoic acid,
(E)-3-[2-n-butyl-1-{(2-chlorophenyl)methyl}-1H-imidazol-5-yl]-2-(5-methyl-2-thienyl)methyl-2-propenoic acid,
(E)-3-[2-n-butyl-1-{(4-carboxyphenyl)methyl}-1H-imidazol-5-yl]-2-(2-thienyl)methyl-2-propenoic acid,
(E)-3-[2-n-butyl-1-{(2-nitrophenyl)methyl}-1H-imidazol-5-yl]-2-(2-thienyl)methyl-2-propenoic acid,
(E}-3-[2-n-butyl-1-{(2-cyanophenyl)methyl}-1H-imidazol-5-yl]-2-(2-thienyl)methyl-2-propenoic acid,
(E)-3-[2-n-butyl-1-{(4-methoxy-3-methylphenyl}methyl}-1H-imidazol-5-yl]-2-(2-thienyl)methyl-2-propenoic acid,
(E)-3-[2-n-butyl-1-{(2-chlorophenyl)methyl}-1H-imidazol-5-yl]-2-(5-methoxy-2-thienyl)methyl-2-propenoic acid,
(E)-3-[2-n-butyl-1-{(2,3-dichlorophenyl)methyl}-1H-imidazol-5-yl]-2-(2-thienyl)methyl-2-propenoic acid,
(E)-3-[2-n-butyl-1-{(4-carboxy-2-chlorophenyl)methyl}-1H-imidazol-5-yl]-2-(2-thienyl)methyl-2-propenoic acid,
(E)-3-[2-n-butyl-1-{(4-carboxy-3-chlorophenyl)methyl}-1H-imidazol-5-yl]-2-(2-thienyl)methyl-2-propenoic acid,
(E)-3-[2-n-hexyl-1-{4-carboxyphenyl}methyl}-1H-imidazol-5-yl]-2-(2-thienyl)methyl-2-propenoic acid,
(E)-3-[2-n-butyl-1-{(4-carbomethoxyphenyl)methyl}-1H-imidazol-5-yl]-2-(2-thienyl)methyl-2-propenoic acid, and
(E)-3-[2-n-butyl-1-{(2-trifluoromethylphenyl)-methyl}-1H-imidazol-5-yl]-2-(2-thienyl)methyl-2-propenoic acid; or a pharmaceutically acceptable salt thereof.

The invention also relates to pharmaceutical compositions comprising a pharmaceutical carrier and an effective amount of a compound of Formula (I).

Also included in the present invention are methods for antagonizing angiotensin II receptors which comprises administering to a subject in need thereof an effective amount of a compound of Formula (I). methods of producing antihypertensive activity and methods of treating congestive heart failure, glaucoma, and renal failure by administering these compounds are also included in this invention.

The compounds of this invention are prepared by procedures described herein and illustrated by the examples. Reagents, protecting groups and functionality on the imidazole and other fragments of the molecule must be consistent with the proposed chemical transformations. Steps in the synthesis must be compatible with the functional groups and the protecting groups on the imidazole and other parts of the molecule.

The starting materials, 2-R²X-imidazole, are known to the art (J. Org. Chem. 45:4038, 1980) or are synthesized by known procedures. For example, imidazole is converted to 2-n-butylimidazole by reacting imidazole with triethylorthoformate and p-toluenesulfonic acid to give 1-diethoxyorthoamide imidazole and then treating with n-butyl lithium to give the 2-lithium derivative of the orthoamide and alkylating with n-butyl iodide in a suitable solvent, such as tetrahydrofuran (THF).

The following procedure is useful for the preparation of compounds of Formula (I) particularly where R¹ is 2-chlorophenyl, R² is n-butyl, R³ is hydrogen, R⁴ is hydrogen, R⁵ is as described in Formula (I), R⁶ is COOH.

The 1-R¹CH₂-group is incorporated onto the 2-R²X-imidazole by known procedures, for example, by reaction with an R¹-CH₂ halide, mesylate or acetate, such as 2-chlorobenzyl bromide, in a suitable solvent, such as dimethylformamide (DMF), in the presence of a suitable acid acceptor, such as sodium alkylate, potassium or sodium carbonate, or a metal hydride, preferably sodium hydride at a reaction temperature of 25°C to 100°C, preferably 50°C. The resulting 1-R¹CH₂-2-R²X imidazole is hydroxymethylated in the 5-position, for example, by reacting with formaldehyde in the presences of sodium acetate in acetic acid to provide the 1-R¹CH₂-2-R²X-5-hydroxymethylimidazole intermediates.

Alternatively, the 1-R¹CH₂-2-R²X-5-hydroxymethylimidazole intermediates are prepared by reacting an imido ether, R²-C(=NH)-O-alkyl, such as valeramidine methyl ether, with dihydroxyacetone in liquid ammonia under pressure to give 2-R²-5-hydroxymethylimidazole. This intermediate is reacted with acetic anhydride to give 1-acetyl-5-acetoxymethyl-2-R²-imidazole. The diacetate intermediate is N-alkylated, for example, using 2-chlorobenzyl triflate and the resulting 1-R¹CH₂-2-R²-5-acetoxymethylimidazole is treated with aqueous base, such as 10% sodium hydroxide solution, to give the 1-R¹CH₂-2-R²-5-hydroxymethylimidazole intermediate.

Alternatively, the 2-R¹S-imidazole compounds are prepared by the following procedure. Benzylamines, substituted by one to three substituents selected from halo, C₁₋₄alkyl, C₁₋₄alkoxy, CN, NO₂, CF₃, CO₂C₁₋₆alkyl, SC₁₋₄alkyl, or SO₂C₁₋₄alkyl, are alkylated with a C₁₋₆alkyl chloroacetate, for example methyl chloroacetate, in the presence of a base, such as triethylamine, in a suitable solvent, such as dimethylformamide. The resulting alkylaminoalkyl ester compounds are N-formulated with formic acid in the presence of a suitable solvent, such as xylenes, followed by C-formulation of the carbon alpha to both the amino and the ester groups. Reaction of this intermediate with acidic thiocyanate, preferably potassium thiocyanate, in an inert organic solvent, such as C₁₋₄alkyl alcohol produces 1-RCH₂-2-mercapto-5-alkanoate ester imidazole compounds. The free thio group of the ester imidazole is reacted with a halo-R¹⁰ compound, wherein R¹⁰ is C₂₋₁₀alkyl, C₃₋₁₀alkenyl, C₃₋₁₀alkynyl, C₃₋₆cycloalkyl or an optionally substituted (CH₂)₀₋₈Ph, preferably propyl bromide, in the presence of a suitable base, such as sodium carbonate, in an appropriate solvent, such as ethyl acetate. The ester is reduced to the hydroxymethyl-imidazole intermediate by reduction with a suitable reagent, preferably diisobutyl aluminum hydride, in an appropriate solvent, such as tetrahydrofuran, at a temperature of -78°C to 25°C, preferably at less than -10°C.

The hydroxymethyl group of the hereinbefore prepared intermediate is oxidized to an aldehyde by treatment with a suitable reagent, such as anhydrous chromic acid-silica gel in tetrahydrofuran or, preferably, with activated manganese dioxide, in a suitable solvent, such as benzene or toluene, or preferably methylene chloride, at a temperature of 25°C to 140°C, preferably at 25°C. The 1-R¹CH₂-2-R²X-imidazol-5-carboxaldehydes are reacted with an appropriate phosphonate, such as those listed in Table I (Examples 2-5). The phosphonates are prepared, for example, from trialkyl phosphonoacetates by alkylation with an appropriate halide, mesylate or acetate in the presence of a suitable base, such as sodium hydride, in a suitable solvent, preferably glyme at a reaction temperature of 25°C to 110°C, preferably at 55°C, to provide, for example, the phosphonates listed in Table I. The reaction of the imidazol-5-carboxaldehydes with the phosphonates is performed in the presence of a suitable base, such as a metal alkoxide, lithium hydride or preferably sodium hydride, in a suitable solvent, such as ethanol, methanol, ether, dioxane, tetrahydmfuran, or preferably glyme, at a reaction temperature of 10°C to 50°C, preferably at 25°C, to provide a variable mixture of trans and cis, e.g., (E) and (Z), 1-R¹CH₂-2-R²X-5-CH=C(R⁵)-(COO-alkyl)-imidazoles. These isomers are readily separated by chromatography over silica gel in suitable solvent systems, preferably hexane in ethyl acetate mixtures. The esters are hydrolyzed to the acids, 1-R¹-CH₂-2-R²X-5-CH=C(R⁵)COOH-imidazoles, using bases, such as potassium hydroxide, lithium hydroxide or sodium hydroxide, in a suitable solvent system, such as, for example, aqueous alcohols or diglyme. The trans and cis structures of the acids are readily determined by NMR by the NOE protocol, as well as by the biological activities since, generally, the trans (E) isomeric acids are the more potent isomers.

Alternatively, the 1-R¹CH₂-2-R²X-imidezol-5-carboxaldehydes are prepared by the following procedure. Starting 2-R²X-imidazol-4-carboxaldehydes are reacted with an N-alkylating protecting reagent, such as chloromethyl pivalate (POM-C1), in the presence of a base, such as dimethylformamide, at a temperature of 20°C to 50°C, preferably at 25°C, to give N-alkylation (e.g., POM-derivanon) on the least hindered nitrogen atom of the imidazole nucleus. The 1-R¹CH₂-group is incorporated onto the imidazole by N-alkylation of the above prepared aldehyde with a halomethylbenzene compounds, such as methyl 4-bromomethyl-3-chlorobenzoate, at a temperature of 80°C to 125°C, preferably at 100°C. The protecting group on the 3-nitrogen of the imidazole ring is removed by base hydrolysis, for example using a biphasic mixture of ethyl acetate and aqueous sodium carbonate, to give 1-R¹CH₂-2-R²X-imidazole-5-carboxaldehyde compounds. The Formula (I) compounds can be prepared from these 5-carboxaldehyde compounds by the methods described above.

Compounds of Formula (I), wherein R⁶ is COOH, R¹, R², R³, R⁴ and R⁵ are as described in Formula (I) are also prepared by the following procedure.

The 2-R²X-imidazole starting materials are reacted with trimethylsilylethoxymethyl (SEM) chloride to give 1-(trimethylsilyl) ethoxymethyl-2-R²-imidazole. The reaction is carried out, for example, in the presence of sodium hydride in a solvent such as dimethylformamide, The 5-tributyltin derivatives are prepared by lithiation with, for example, butyllithium in a suitable solvent, preferably diethyl ether, followed by treatment of the lithio imidazole derivative with a tributyltin halide, preferably tri-N-butyltin chloride, at -10°C to 35°C, preferably at 25°C. The 1-SEM-2-R²-5-tributyltinimidazole is coupled with an α,β-unsaturated acid ester having a leaving group on the β-position, such as a halide or trifluoromethanesulfonyloxy group, for example, BrCR⁴=C(R⁵)(COOalkyl), in the presence of a phosphine ligand, such as bis(diphenylphosphino)propane, or triphenylphosphine and a palladium (II) compound, or preferably tetrakis(triphenylphosphine)-palladium(0), with or without a base, such as tributylamine, at a temperature of 50°C to 150°C, preferably at 120°C. Both the (E) and (Z) olefinic isomers are prepared by this procedure, and the isomeric esters are readily separated by chromatography over silica gel. The 1-SEM group from the (E) and (Z) isomers is hydrolyzed with acid, for example, aqueous hydrochloric, in a suitable alcoholic solvent, such as methanol or ethanol, and the 1-unsubstituted imidazole derivatives are converted to the 1-t-butoxycarbonyl (t-BOC) imidazoles with di-t-butyl dicarbonate (Hoppe-Seyler's Z. Physiol. Chem., (1976), 357, 1651). The t-BOC esters are alkylated and hydrolyzed with, for example, 2-chlorobenzyl-O-triflate in the presence of a suitable base, preferably diisopropylethylamine, in a suitable solvent, preferably methylene chloride, to afford the 1-(2-chlorophenyl)methylimidazole derivatives (esters). The (E) and (Z) isomers are hydrolyzed to the (E) and (Z) acids by the method described above.

Compounds of Formula (I) are also prepared by the following procedure. The 1-R¹CH₂-2-R²X-imidazole-5-carboxaldehydes, prepared as described above, are reacted with a substituted half-acid, half-ester derivative of a malonate, such as ethyl 2-carboxy-3-(2-thienyl)propionate, in the presence of a base, such as piperidine, in a suitable solvent, such as toluene, at a temperature of 80°C to 110°C, preferably at 100°C. The resulting 1-R¹CH₂-2-R²X-5-CH=C(R⁵)COO alkylimidazoles are hydrolyzed to the corresponding Formula (I) acid compounds by alkaline hydrolysis as described above.

Compounds of Formula (I) in which R³ is H are prepared as follows. The 1-R¹-CH₂-2-R²X-imidazol-5-carboxaldehydes are treated with the lithium derivatives of a substituted ethyl or methyl ester. These lithio derivatives are prepared from the reaction of lithium diisopropylamide in a suitable solvent, preferably tetrahydrofuran, with an acid ester, such as ROOC-CH₂-Y-(2-thienyl), to generate the α-lithio derivatives at -78°C to -10°C, preferably at -78°C, which are then treated with the imidazol-carboxaldehyde. The intermediate β-hydroxy group of the imidazol ester is converted to a mesylate or an acetate and the mesylate, or preferably the acetate, is heated in a suitable solvent, such as toluene, with one to two equivalents of 1,8-diazo-bicyclo[5.4.0]undec-7-ene, at 50 to 110°C, preferably at 80°C, to afford ester compounds of Formula (I) such as 3-(imidazol-5-yl)-2-(2-thienyl)methyl-2-propenoic acid esters. The (E) isomer is the predominate olefinic isomer. The acids are prepared from the esters by the method described above.

Compounds of Formula (I), wherein R¹ is 2-chlorophenyl, R² is n-butyl, R³ is H, R⁴ is H, R⁵ is heterocyclic or a substituted heterocyclic group as described in Formula (I) and R⁶ is COOH, may be prepared by heating 1-R¹-CH₂-2-R²X-imidazol-5-carboxaldehydes at 50°C to 180°C, preferably at 140°C, with an appropriate substituted heterocyclic acetic acid and with acetic anhydride and potassium carbonate to provide unsaturated acids of formula (I) such as 3-[2-n-butyl-1-(2-chlorophenyl)methyl-1H-imidazol-5-yl]-2-R⁵-2-propenoic acid. The trans olefinic acid is the principal product.

Compounds of Formula (I) in which the R¹ substituent is substituted by hydroxy are formed from Formula (I) compounds in which the R¹ group is substituted by C₁-C₄alkoxy using an ether-cleaving reagent, such as boron tribromide or hydrobromic acid.

Compounds of Formula (I) in which the R¹ substituent is substituted by carboxy are formed from Formula (I) compounds in which the R¹ group is substituted by CO₂C₁-C₄alkyl using basic hydrolysis or ethanol, or using acidic hydrolysis, such as aqueous hydrochloric acid.

Compounds of Formula (I) in which the R¹ substituent is substituted by a tetrazol-5-yl group are prepared from the corresponding carboxy compounds. For example, Formula (I) acid compounds are reacted with a halogenating agent, such as thionyl chloride, in a suitable solvent, for example benzene, to give the corresponding acid halide compounds. The acid halides are then converted to primary amide compounds in a reaction with concentrated ammonia. Subsequent dehydration of the amides with oxalyl chloride/dimethylformamide in acetonitrile/dimethylformamide yields and the nitrile compounds, which are the immediate precursors to the Formula (I) tetrazole compounds. Tetrazole formation is accomplished by reacting the nitrites with azide, preferably aluminum azide prepared in situ by the reaction of sodium azide with aluminum chloride, in a suitable solvent, for example tetrahydrofuran. The Formula (I) compounds in which R⁶ is -CO₂H are prepared from these Formula (I) tetrazole ester compounds by basic hydrolysis as described above.

Pharmaceutically acceptable acid addition salts of compounds of Formula (I) are formed with appropriate organic or inorganic acids by methods known in the art. For example, the base is reacted with a suitable inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Representative examples of suitable acids are maleic, fumaric, benzoic, ascorbic, pamoic, succinic, bismethylene-salicyclic, methanesulfonic, ethanedisulfonic, acetic, propionic, tartaric, salicyclic, citric, gluconic, aspartic, stearic, palmitic, itaconic, glycolic, p-aminobenzoic, glutamic, benzenesulfonic, hydrochloric, hydrobromic, sulfuric, cyclohexyl-sulfamic, phosphoric and nitric acids.

Pharmaceutically acceptable base addition salts of compounds of Formula (I) in which R⁸ is H are prepared by known methods from organic and inorganic bases, including nontoxic alkali metal and alkaline earth bases, for example, calcium, lithium, sodium, and potassium hydroxide; ammonium hydroxide, and nontoxic organic bases, such as triethylamine, butylamine, piperazine, and (trihydroxymethyl)-methylamine.

Angiotensin II antagonist activity of the compounds of Formula (I) is assessed by in vitro and in vivo methods. In vitro antagonist activity is determined by the ability of the compounds to compete with ¹²⁵I-angiotensin II for binding to vascular angiotensin It receptors and by their ability to antagonize the contractile response to angiotensin II in the isolated rabbit aorta. In vivo activity is evaluated by the efficacy of the compounds to inhibit the pressor response to exogenous angiotensin II in conscious rats and to lower blood pressure in a rat model of resin dependent hypertension.

### Binding

The radioligand binding assay is a modification of a method previously described in detail (Gunther et al., Circ. Res. 47:278, 1980). A particular fraction from rat mesenteric arteries is incubated in Tris buffer with 80 pM of ¹²⁵I-angiotensin II with or without angiotensin II antagonists for 1 hour at 25°C. The incubation is terminated by rapid filtration and receptor bound ¹²⁵I-angiotensin II trapped on the filter is quantitated with a gamma counter. The potency of angiotensin II antagonists is expressed as the IC₅₀ which is the concentration of antagonist needed to displace 50% of the total specifically bound angiotensin II. Exemplary of the IC₅₀ of compounds of the invention (E isomers) is about 0.1 nM to about 100 µM.

### Aorta

The ability of the compounds to antagonize angiotensin II induced vasoconstriction is examined in the rabbit aorta. Ring segments are cut from the rabbit thoracic aorta and suspended in organ baths containing physiological salt solution. The ring segments are mounted over metal supports and attached to force displacement transducers which are connected to a recorder. Cumulative concentration response curves to angiotensin II are performed in the absence of antagonist or following a 30-minute incubation with antagonist. Antagonist disassociation constants (K_{B}) are calculated by the dose ratio method using the mean effective concentrations. Exemplary of the K_{B} of compounds of the invention (E isomers) is about 0.1 nM to about 30 µM.

### Inhibition of pressor response to angiotensin II in conscious rats

Rats are prepared with indwelling femoral arterial and venous catheters and a stomach tube (Gellai et al., Kidney Int. 15:419, 1979). Two to three days following surgery the rats are placed in a restrainer and blood pressure is continuously monitored from the arterial catheter with a pressure transducer and recorded on a polygraph. The change in mean arterial pressure in response to intravenous injections of 250 mg/kg angiotensin II is compared at various time points prior to and following the administration of the compounds intravenously or orally at doses of 0.1 to 300 mg/kg. The dose of compound needed to produce 50% inhibition of the control response to angiotensin II (IC₅₀) is used to estimate the potency of the compounds. The IC₅₀ of (E)-3-[2-n-butyl-1-{(2-chlorophenyl)methyl}-1H-imidazol-5-yl]-2-(2-thienyl)methyl-2-propenoic acid is 3.60 mg/kg i.v. and 44.00 mg/kg orally.

### Antihypertensive activity

The antihypertensive activity of the compounds is measured by their ability to reduce mean arterial pressure in conscious rats made renin-dependent hypertensive by ligation of the left renal artery (Cangiano et al., J. Pharmacol. Exp. Ther. 208:310, 1979). Renal artery ligated rats are prepared with indwelling catheters as described above. Seven to eight days following renal artery ligation, the time at which plasma renin levels are highest, the conscious rats are placed in restrainers and mean arterial pressure is continuously recorded prior to and following the administration of the compounds intravenously or orally. The dose of compound needed to reduce mean arterial pressure by 30 mm Hg (IC₅₀) is used as an estimate of potency. The IC₅₀ of (E)-3-[2-n-butyl-1-{(2-chlorophenyl)methyl}-1H-imidazol-5-yl]-2-(2-thienyl)methyl-2-propenoic acid is 1.80 mg/.kg i.v. and 8.0 mg/kg orally.

The intraocular pressure lowering effects employed in this invention may be measured by the procedure described by Watkins, et al., J. Ocular Pharmacol., 1 (2):161-168 (1985).

The compounds of Formula (I) are incorporated into convenient dosage forms, such as injectable preparations, or for orally active compounds, capsules or tablets. Solid or liquid pharmaceutical carriers are employed. Solid carriers include starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid, such as an ampoule, or an aqueous or nonaqueous liquid suspension.

For topical ophthalmologic administration, the pharmaceutical compositions adapted include solutions, suspensions, ointments, and solid inserts. Typical pharmaceutically acceptable carriers are, for example, water, mixtures of water and water-miscible solvents such as lower alkanols or vegetable oils, and water soluble ophthalmologically acceptable non-toxic polymers, for example, cellulose derivatives such as methyl cellulose. The pharmaceutical preparation may also contain non-toxic auxiliary substances such as emulsifying, preserving wetting, and bodying agents, as for example, polyethylene glycols; antibacterial components, such as quaternary ammonium compounds; buffering ingredients, such as alkali metal chloride; antioxidants, such as sodium metabisulfite; and other conventional ingredients, such as sorbitan monolaurate.

Additionally, suitable ophthalmic vehicles may be used as carrier media for the present purpose including conventional phosphate buffer vehicle systems.

The pharmaceutical preparation may also be in the form of a solid insert. For example, one may use a solid water soluble polymer as the carrier for the medicament. Solid water insoluble inserts, such as those prepared from ethylene vinyl acetate copolymer, may also be utilized.

The pharmaceutical preparations are made following conventional techniques of a pharmaceutical chemist involving mixing, granulating, and compressing, when necessary, for tablet forms, or mixing, filling and dissolving the ingredients, as appropriate, to give the desired oral, parenteral, or topical products.

Doses of the compounds of Formula (I) in a pharmaceutical dosage unit as described above will be an efficacious, nontoxic quantity selected from the range of 0.01 - 200 mg/kg of active compound, preferably 1 - 100 mg/kg. The selected dose is administered to a human patient in need of angiotensin II receptor antagonism from 1-6 times daily, orally, rectally, topically, by injection, or continuously by infusion. Oral dosage units for human administration preferably contain from 1 to 500 mg of active compound. Preferably, lower dosages are used for parenteral administration. Oral administration, at higher dosages, however, also can be used when safe and the active compound in an amount selected from 0.0001 to 0.1 (w/v%), preferably from 0.0001 to 0.01. As a topical dosage unit form, an amount of active compound from between 50 ng to 0.05 mg, preferably 50 ng to 5 µg, is applied to the human eye.

The method of this invention of antagonizing angiotensin II receptors in mammals, including humans, comprises administering to a subject in need of such antagonism an effective amount of a compound of Formula (I). The method of this invention of producing antihypertensive activity and the method of treating congestive heart failure, glaucoma, and renal failure comprise administering a compound of Formula (I) to a subject in need thereof an effective amount to produce said activity.

Contemplated equivalents of Formula (I) compounds are compounds otherwise corresponding thereto wherein substituents have been added to any of the unsubstituted positions of the Formula (I) compounds provided such compounds have the pharmaceutical utility of Formula (I) compounds.

The following examples illustrate preparation of compounds and pharmaceutical compositions of this invention. The examples are not intended to limit the scope of this invention as described hereinabove and as claimed below.

### Example 1

### (E)-3-[2-n-Butyl-1-{(2-chlorophenyl)methyl}-1H-imidazol-5-yl]-2-(2-thienyl)methyl-2-propenoic Acid

### (i) 2-n-butyl-1-(2-chloro-phenyl)methyl-1H-imidazole

Imidazole was converted to the 1-diethoxyorthoamide derivative by the method of Curtis and Brown, J. Org. Chem., (1980), 45, 20. Imidazole (12.8 g, 0.19 mol) and 118.4 g (0.8 mol) of triethylorthaformate were reacted in the presence of 1 g of p-toluenesulfonic acid to give 20.6 (61%), bp 65-70°C (0.1 mm) of 1-diethoxyorthoamide imidazole. This product (24.0 g, 0.14 mol) was dissolved in dry tetrahydrofuran (250 mL), cooled to -40°C and n-butyl lithium (0.14 mol, 56.4 mL of 2.5 M in hexane) was added at -40°C to -35°C. After 15 minutes n-butyl iodide (31.1 g, 0.169 mol) was added at -40°C, and the reaction was stirred overnight at ambient temperature. The reaction was partitioned between ether and 0.3 N hydrochloric acid, and the organic layer was repeatedly extracted with dilute hydrochloric acid. The combined aqueous extracts were neutralized with sodium bicarbonate solution, extracted with methylene chloride, dried over magnesium sulfate and concentrated. A flash distillation on a Kugelrohr apparatus provided 14.8 g (85%) of 2-n-butylimidazole.

2-n-Butylimidazole (9.7 g, 0.078 mol) was dissolved in methanol (50 mL) and added dropwise to a solution of sodium methoxide (from sodium hydride (2.31 g, 0.0934 mol) in methanol (250 mL)). After one hour the solution was evaporated to dryness, and the sodium salt was taken up in dry dimethylformamide (150 mL) and 2-chlorobenzyl bromide (16.3 g, 0.079 mol) was added. The mixture was heated at 50°C for 17 hours under argon, poured onto ice water and the product was extracted into ethyl acetate. The extract was washed, dried, and concentrated to give 18.5 g of crude product which was chromatographed over silica gel with 2:1 ethyl acetate/hexane to provide 11.9 g (61%) of 2-n-butyl-1-(2-chlorophenyl)methyl-1H-imidazole as an oil. Thin layer chromatography on silica gel with 4:1 ethyl acetate/hexane gave an R_{f} value of 0.59.

### (ii) 2-n-butyl-1-(2-chlorophenyl)methyl-5-hydroxymethyl-1H-imidazole

Method 1 A mixture of 2-n-butyl-1-(2-chlorophenyl)methyl-1H-imidazole (95.5 g, 0.384 mol), 37% formaldehyde (500 mL), sodium acetate (80 g) and acetic acid (60 mL) was heated to reflux for 40 hours under argon. The reaction was concentrated in vacuo, and the residue was stirred with 500 mL of 20% sodium hydroxide solution for 4 hours, diluted with water and extracted with methylene chloride. The extract was washed, dried, and concentrated. The crude product (117 g) was flash chromatographed over 600 g of silica gel with a gradient of ethyl acetate to 10% of methanol in ethyl acetate to give 8.3 g of starting material, 24.5 g of a mixture of starting material and product, and 44 g (41%) of 2-n-butyl-1-(2-chlorophenyl)-methyl-5-hydroxymethyl-1H-imidazole; mp 86-88°C (from ethyl acetate). Further elution provided the bis (4,5-hydroxymethyl) derivative; mp 138-140°C (from ethyl acetate).
Method 2 A mixture of valeramidine methyl ether hydrochloride (250 g, 1.66 mol) and dihydroxyacetone (150 g, 0.83 mol) dissolved in liquid ammonia was allowed to stand overnight at room temperature in a pressure vessel, and then heated at 65°C for 4 hours at 375 psi. The ammonia was allowed to evaporate, and the residue was dissolved in methanol (3L). The resulting slurry was refluxed with added acetonitrile (1L). The solution was decanted from the solid ammonium chloride while hot. This procedure was repeated, and the combined acetonitrile extracts were treated with charcoal, filtered hot and the filtrate was concentrated in vacuum to give the dark oil, 2-n-butyl-5-hydroxymethylimidazole (253 g, 1.63 mol, 98%).

This crude alcohol (253 g) was treated with acetic anhydride (400 mL) at -15°C and then was allowed to warm to ambient temperature with stirring, and then stirred an additional 19 hours. The acetic anhydride was evaporated at reduced pressure, the residue taken up in methylene chloride, and the organic phase was washed with 5% sodium bicarbonate solution and water. The extract was dried over sodium sulfate and concentrated to give 323 g (83%) of 1-acetyl-4-acetoxymethyl-2-n-butylimidazole.

This diacetate was N-alkylated by the following procedure. To a solution of triflic anhydride (120 mL, 0.71 mol) in methylene chloride (200 mL) at -78°C under argon was added a solution of diisopropyl ethylamine (128 mL, 0.73 mol) and 2-chlorobenzyl alcohol (104 g, 0.72 mol) in methylene chloride (350 mL) over a period of 20 minutes. After being stirred an additional 20 minutes at -78°C, this solution was then treated with 1-acetyl-4-acetoxymethyl-2-n-butylimidazole (146 g, 0.61 mol) dissolved in methylene chloride (300 mL) over a 20-minute interval. The mixture was then stirred at ambient temperature for 18 hours and the solvents were evaporated, The residual 2-n-butyl-5-acetoxymethyl-1-(2-chlorophenyl)methyl-1H-imidazole was used without purification for the hydrolysis of the acetate group.

A solution of crude 2-n-butyl-5-acetoxymethyl-1-(2-chlorophenyl)methyl-1H-imidazole (250 g) in methanol (200 mL) was treated with 10% sodium hydroxide solution (700 mL) and the mixture was heated on a steam bath for 4 hours. After cooling, methylene chloride was added, the organic phase was separated, washed with water, dried and concentrated. The residue was dissolved in ether, cooled, and seeded to give the crude product. Recrystallization from ethyl acetate gave 176 g of 2-n-butyl-1-(2-chlorophenyl)methyl-5-hydroxymethyl-1H-imidazole; mp 86-88°C. This material was identical in all respects to the product prepared by Method 1.

### (iii) 2-n-butyl-1-(2-chlorophenyl)methyl-1H-imidazol-5-carboxaldehyde

A solution of 2-n-butyl-1-(2-chlorophenyl)methyl-5-hydroxymethyl-1H-imidazole (5.4 g, 0.0194 mol) in toluene (25 mL) was added to a suspension of activated manganese dioxide (27 g) in methylene chloride (325 mL). The suspension was stirred at room temperature for 17 hours. The solids were filtered and the filtrate concentrated and flash chromatographed over silica gel with 6:4 hexane/ethyl acetate to afford 4.16 g (78%) of 2-n-butyl-1-(2-chlorophenyl)methyl-1H-imidazol-5-carboxaldehyde, as an oil. NMR and IR were consistent with the structure.

### (iv) (E)-3-[2-n-butyl-1-{(2-chloropheny)methyl}-1H-imidazol-5-yl]-2-(2-thienyl)methyl-2-propenoic acid

### Method A (a) trimethyl 3-(2-thienyl)-2-phosphonopropionate

To a solution of 2-thiophenemethanol (2.28 g, 0.02 mol) in carbon tetrachloride (25 mL) was added triphenylphosphine (6.81 g, 0.026 mol), and the solution was refluxed for 3 hours. The cooled reaction mixture was diluted with hexane (60 mL), chilled and filtered. The concentrated filtrate (4.6 g) was flash chromatographed over silica gel with 7:3 hexane/ethyl acetate to provide 2-chloromethylthiophene (1.52 g, 57%) as an oil.

A suspension of sodium hydride (0.271 g, 11.3 mmol) in dry glyme (40 mL) under argon was treated dropwise with trimethyl phosphonoacetate (1.87 g, 10.3 mmol) in glyme (5 mL). The resulting mixture was stirred at room temperature for 1.5 hours. Then 2-chloromethyl-thiophene (1.5 g, 11.3 mmol) was added, and the mixture was stirred at 65°C for 18 hours. The reaction was partitioned between water and ethyl acetate, and the organic layer was washed with water and brine, dried with anhydrous magnesium sulfate and concentrated to 1.9 g of an oil. This was chromatographed over silica gel 4:1 ethylacetate/hexane to afford 800 mg (28%) of trimethyl 3-(2-thienyl)-2-phosphonopropionate.

### (b) methyl (E)-3-[2-n-butyl-1-{(2-chlorophenyl)methyl}-1H-imidazol-5-yl-2-(2-thienyl)methyl-2-propenoate

To a suspension of sodium hydride (69 mg, 2.87 mmol) in glyme (5 mL) was added dropwise a solution of trimethyl 3-(2-thienyl)-2-phosphonopropionate in glyme (3 mL) under an atmosphere of argon. When the gas evolution had subsided, the mixture was heated to 50°C for 15 minutes. A solution of 2-n-butyl-1-(2-chlorophenyl)methyl-1H-imidazol-5-carboxaldehyde (0.53 g, 1.92 mmol) in glyme (3 mL) was added, and the mixture was stirred at 60-65°C for 5 hours. The cooled reaction was partitioned between water and ethyl acetate, and the organic layer was washed with water, dried, concentrated and flash chromatographed over silica gel to give 336 mg (41%) of methyl (E)-3-[2-n-butyl-1-[(2-chlorophenyl)methyl]-1H-imidazol-5-yl[-2-(2-thienyl)methyl-2-propenoate as an oil whose NMR was entirely consistent with the trans or E form of the olefin.

### (c) (E)-3-[2-n-butyl-1-{(2-chlorophenyl)methyl}-1H-imidazol-5-yl]-2-(2-thienyl)methyl-2-propenoic acid

A solution of methyl (E)-3-[2-n-butyl-1-[(2-chlorophenyl)methyl]-1H-imidazol-5-yl]-2-(2-thienyl)methyl-2-propenoate (336 mg, 0.783 mmol) in ethanol (10 mL) was treated with 10% sodium hydroxide solution (4 mL), and the solution was stirred for 3 hours at 25°C. The pH was adjusted to 5 and a solid precipitated. The mixture was diluted with water, cooled and filtered to provide 309 mg of solid. A crystallization from ethyl acetate gave 195 mg (60%) of (E)-3-[2-n-butyl-1-[(2-chlorophenyl)methyl]-1H-imidazol-5-yl]-2-(2-thienyl)methyl-2-propenoic acid; mp 177-179°C.

### Method B (a) methyl 3-[2-n-butyl-1-{(2-chlorophenyl)methyl}-1H-imidazol-5-yl]-3-hydroxy-2-(2-thienyl)methylpropanoate

To a solution of diisopropylamine (1.96 g, 0.0194 mol) in dry tetrahydrofuran (40 mL) held at -78°C under argon was added n-butyl lithium (7.3 mL, 0.0183 mol of 2.5 M in toluene), and the mixture was stirred for 10 minutes. Then, methyl 3-(2-thienyl)propanoate (2.83 g, 0.0166 mol) in tetrahydrofuran (2 mL) was added, and the mixture was stirred for 30 minutes at -78°C. A solution of 2-n-butyl-1-(2-chlorophenyl)methyl-1H-imidazol-5-carboxaldehyde (3 g, 0.0111 mol) in tetrahydrofuran (4 mL) was added, and the resulting mixture was stirred at -78°C for 30 minutes. The reaction was partitioned between saturated ammonium chloride solution and ether, the organic extract was washed with brine, dried over anhydrous magnesium sulfate and concentrated to 6.67 g of crude product. This was flash chromatographed over 70 g of silica gel with 4:1 ethyl acetate/hexane to provide 4.03 g (81%) of methyl 3-[2-n-butyl-1-(2-chlorophenyl)methyl-1H-imidazol-5-yl]-3-hydroxy-2-(2-thienyl)methyl-propanoate.

### (b) methyl 3-acetoxy-3-[2-n-butyl-1-(2-chlorophenyl)methyl-1H-imidazol-5-yl]-2-(2-thienyl)methylpropanoate

A solution of methyl 3-[2-n-butyl-1-(2-chlorophenyl)methyl-1H-imidazol-5-yl]-3-hydroxy-2-(2-thienyl)-methylpropanoate (4.03 g, 9.02 mmol) in methylene chloride (100 mL) was treated with 4-dimethylaminopyridine (0.386g, 3.16 mmol). Then acetic anhydride (8.5 mL, 9.02 mmol) was added dropwise to the stirred mixture. The mixture was stirred for 18 hours, water (35 mL) was added, the mixture was stirred for 1 hour and then diluted with ether and saturated sodium bicarbonate solution. The ether layer was washed with brine, dried with anhydrous magnesium sulfate and evaporated to give the title 3-acetoxy derivative as an oil (4.37 g, 99%).

### (c) methyl (E)-3-[2-n-butyl-1-{(2-chlorophenyl)methyl}-1H-imidazol-5-yl]-2-(2-thienyl)methyl-2-propenoate

A mixture of methyl 3-acetoxy-3-[2-n-butyl-1-(2-chlorophenyl)methyl-1H-imidazol-5-yl]-2-(2-thienyl)-methylpropanoate (4.36 g, 8.92 mmol) in dry toluene (80 mL) was treated with 1,8-diazabicyclo[5.4.0]undec-7-ene (DBU) (3.2 mL, 21.4 mmol), and the resulting solution was heated at 80°C under argon for 3 hours. The, solvent was evaporated, the residue triturated with ether and activated charcoal was added. After filtration, the filtrate was concentrated to 6.29 g of an oil that was chromatographed over silica gel with 65:35 hexane/ethyl acetate to give 2.89 g (76%) of methyl (E)-3-[2-n-butyl-1-[(2-chlorophenyl)-methyl]-1H-imidazol-5-yl]-2-(2-thienyl)-methyl-2-propenoate whose NMR and TLC (50% ethyl acetate in hexane on silica gel) were identical to the product prepared by Method A.

### (d) (E)-3-[2-n-butyl-1-{(2-chlorophenyl)methyl}-1H-imidazol-5-yl]-2-(2-thienyl)methyl-2-propenoic acid

Basic hydrolysis of this ester (2.88 g, 6.71 mmol) according to Method A (iii) gave 2.59 g (93%) of (E)-3-[2-n-butyl-1-[(2-chlorophenyl)methyl]-1H-imidazol-5-yl]-2-(2-thienyl)methyl-2-propenoic acid; mp 175-177°C that was identical to the product from Method A.

### Examples 2-3

In Table I are listed other examples of alkenoic acids prepared from 2-n-butyl-1-(2-chlorophenyl)methyl-1H-imidazol-5-carboxaldehyde by the methods described in Example 1 (Method A). The reagents and products are shown in Table I.

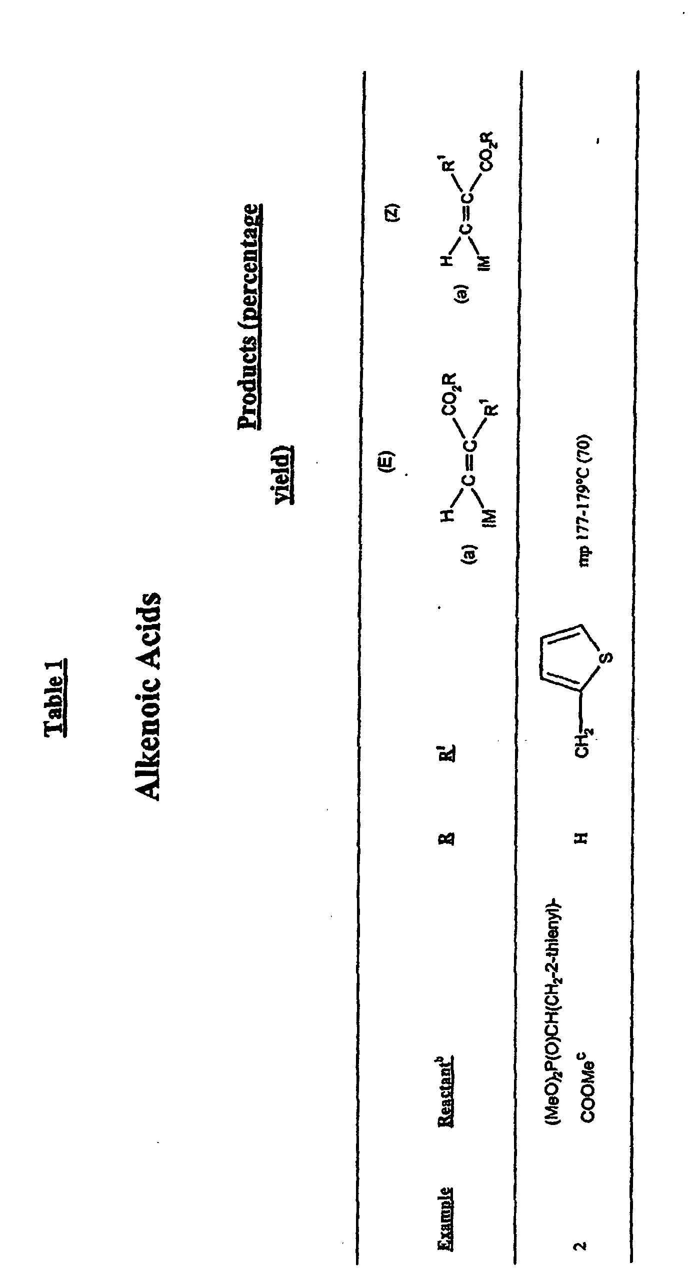

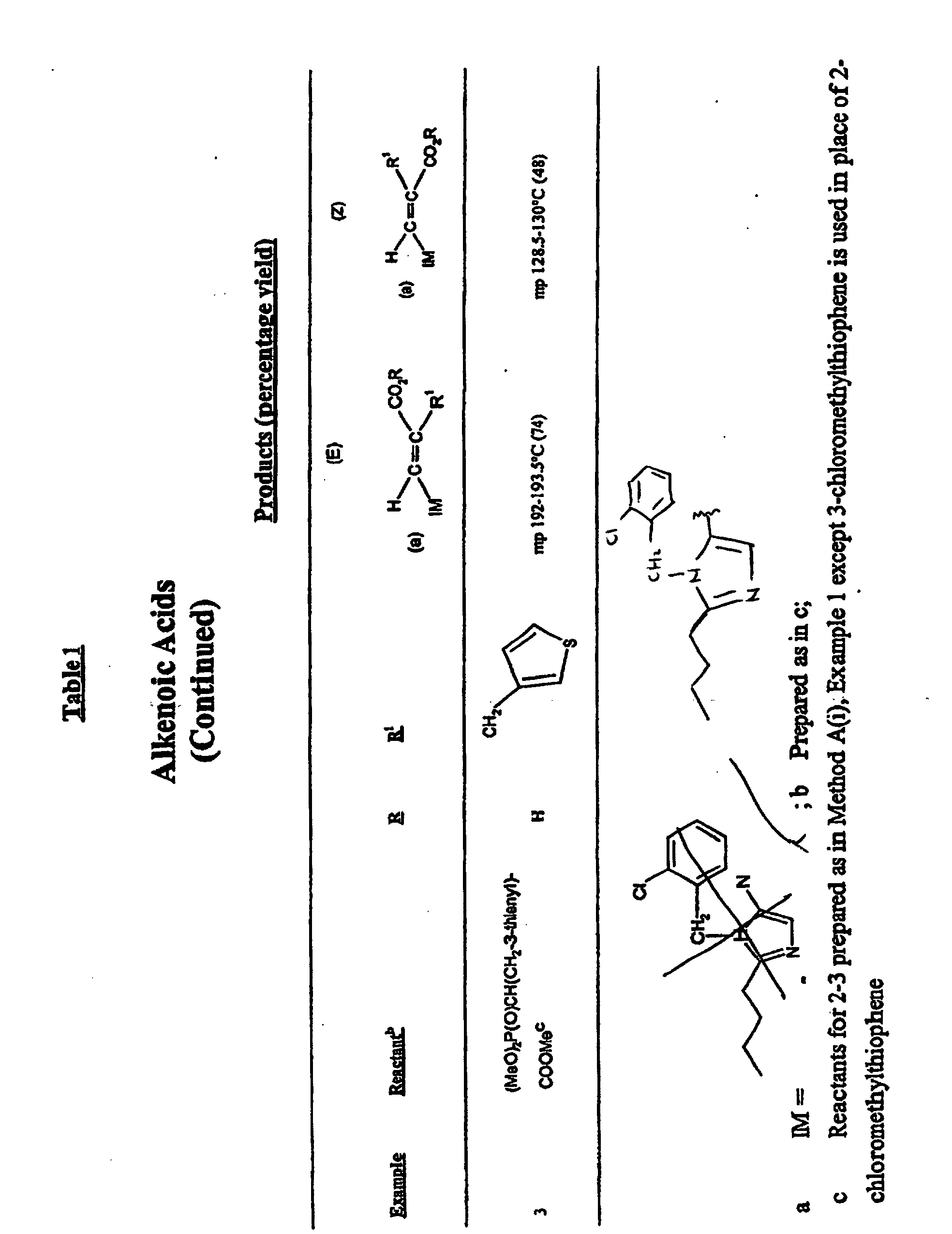

### Example 4

### (E)-3-[2-n-Butyl-1-{(2-chloro-6-fluoro-phenyl)methyl}-1H-imidazol-5-yl]-2-(2-thienyl)methyl-2-propenoic Acid

### (i) 2-n-butyl-1-(2-chloro-6-fluorophenyl)methyl-1H-imidazole

A solution of 2-n-butylimidazole (3.75 g, 0.03 mol) in dry dimethylformamide (4 mL) was added to sodium hydride (0.95 g) in dimethylformamide (18 mL). After the gas evolution subsided, the mixture was stirred one hour under argon and 2-chloro-6-fluorobenzylchloride (5.05 g, 0.031 mol) in dimethylformamide (7 mL) was added to produce an exotherm. The mixture was stirred for 17 hours at ambient temperature, diluted with ice water and extracted with ethyl acetate. The washed, dried, concentrated organic layer provided 7.63 (94%) of the title compound whose NMR was consistent with the structure. This material was used without further purification.

### (ii) 2-n-butyl-1-(2-chloro-6-fluorophenyl)methyl-1H-imidazole-5-carboxaldehyde

The procedures of Example 1(ii-iii) were used. From 7.63 g of crude 2-n-butyl-1-(2-chloro-6-fluorophenyl)methyl-1H-imidazole and proportional amounts of other reagents was obtained 2.8 g of 2-n-butyl-1-(2-chloro-6-fluorophenyl)methyl-5-hydroxymethyl-1H-imidazole after chromatography over silica gel with 3% of methanol in methylene chloride; mp 106-108°C (from ethyl acetate). This material was oxidized with manganese dioxide and worked up as described above to give 0.88 g (63%) of 2-n-butyl-2-(2-chloro-6-fluorophenyl)methyl-1H-imidazole-5-carboxaldehyde; mp 88-90°C (from ethyl acetate).

### (iii) (E)-3-[2-n-butyl-1-{(2-chloro-6-fluorophenyl)methyl}-1H-imidazol-5-yl]-2-(2-thienyl)methyl-2-propenoic acid

The procedure of Example 1, Method A is used. 2-n-Butyl-1-(2-chloro-6-fluorophenyl)methyl-1H-imidazole-5-carboxaldehyde, trimethyl 3-(2-thienyl)-2-phosphono-propionate, sodium hydride and glyme are held at 60°C for 1 hour to give, after chromatography over silica gel with 50% of hexane in ethyl acetate, methyl (E)-[2-n-butyl-1-{(2-chloro-6-fluorophenyl)methyl}-1H-imidazol-5-yl]-2-(2-thienyl)methyl-2-propenoate and corresponding cis or (Z)-isomer. The (E)-isomer is hydrolyzed to afford (E)-3-[2-n-butyl-1-{(2-chloro-6-fluorophenyl)methyl}-1H-imidazol-5-yl]-2-(2-thienyl)methyl-2-propenoic acid.

### Example 5

### (E)-3-[2-n-Butyl-1-{(2-chlorophenyl)methyl}-1H-imidazol-5-yl]-2-(2-thienyl)-2-propenoic Acid

A mixture of 2-n-butyl-1-(2-chlorophenyl)methyl-1H-imidazole-5-carboxaldehyde (2 mmol), 2-thienylacetic acid (2.3 mmol), potassium carbonate (0.91 mmol), and acetic anhydride (1 mL) is heated gradually to 140°C and held at this temperature for 6 hours. The cooled reaction is diluted with water and the solid is separated, triturated several times with ether, and the solid is crystallized to give the title compound.

### Preparation 1

### (E)-3-[2-n-Butyl-1-{(2-chlorophenyl)methyl}-1H-imidazol-5-yl]-2-(4-pyridyl)methyl-2-propenoic Acid

### (i) methyl 3-[2-n-butyl-1-(2-chlorophenyl)methyl-1H-imidazol-5-yl]-3-hydroxy-2-(4-pyridyl)methylpropanoate

To a solution of diisopropylamine (3.58 mL, 25.6 mmol) in dry tetrahydrofuran (50 mL) held at -78°C under argon was added n-butyl lithium (10.2 mL, 25.6 mmol of 2.5 M in toluene), and the mixture was stirred for 10 minutes. Then, methyl 3-(4-pyridyl)propanoate (4.22 g, 25.6 mmol) (prepared by reaction of 4-pyridine carboxaldehyde with trimethyl phosphonoacetate in the presence of sodium hydride in ethylene glycol dimethyl ether, followed by catalytic hydrogenation of the double bond with 10% palladium on carbon at 3 atmosphere of hydrogen in an ethyl acetate solution (98%) to provide the saturated ester) was added in tetrahydrofuran (40 mL) and this mixture was stirred for 30 minutes at -78°C. A solution of 2-n-butyl-1-(2-chlorophenyl)methyl-1H-imidazol-5-carboxaldehyde (5.9 g, 21.3 mmol) in tetrahydrofuran (10 mL) was added and stirring was continued for 30 minutes at -78°C. The reaction was partitioned between saturated ammonium chloride solution and ether, the organic extract was washed with brine, dried over magnesium sulfate, concentrated and flash chromatographed over silica gel with 5% methanol in ethyl acetate to provide 3.32 g (30%) of methyl 3-[2-n-butyl-1-(2-chlorophenyl)-methyl-1H-imidazol-5-yl]-3-hydroxy-2-(4-pyridyl)methyl-propanoate. TLC on silica gel with 5% methanol in ethyl acetate showed a homogenous product with an R_{f} of 0.79.

### (ii) methyl 3-acetoxy-3-[2-n-butyl-1-(2-chlorophenyl)methyl-1H-imidazol-5-yl]-2-(4-pyridyl)propanoate

A solution of methyl 3-[2-n-butyl-1-(2-chlorophenyl)-methyl-1H-imidazol-5-yl]-3-hydroxy-2-(4-pyridyl)-methyl-propanoate (3.32 g, 7.5 mmol) methylene chloride (50 mL), 4-dimethylaminopyridine (150 mg, 1.3 mmol) and acetic anhydride (7.1 mL, 75 mmol) was stirred at ambient temperature for 18 hours. Water (5 mL) was added, the mixture was stirred for 2 hours and then diluted with methylene chloride and 5% sodium bicarbonate solution. The organic phase was washed with 5% sodium bicarbonate solution and brine, dried and concentrated to give 4 g of the crude title compound. TLC on silica gel with 5% methanol ethyl acetate showed essentially one spot material with an R_{f} of 0.86. No starting material was detected. This material was not purified further.

### (iii) methyl (E)-3-[2-n-butyl-1-{(2-chlorophenyl)-methyl}-1H-imidaxol-5-yl]-2-(4-pyridyl)methyl-2-propenoate

A mixture of methyl 3-acetoxy-3-[2-n-butyl-1-(2-chlorophenyl)methyl-1H-imidazol-5-yl]-2-(4-pyridyl)-propenoate (7.5 mmol), toluene (50 mL) and 1,8-diaza-bicyclo[5,4,0]-undec-7-ene (DBU) (3.4 mL, 22.5 mmol) was heated at 90°C for 18 hours under argon. The cooled mixture was diluted with ether, and washed with brine, dried and concentrated to 3.1 g (97%) of the title compound. NMR showed that the trans or E isomer was the primary product.

### (iv) (E)-3-[2-n-butyl-1-{(2-chlorophenyl)methyl}-1H-imidazol-5-yl]-2-(4-pyridyl)-methyl-2-propenoic acid

A solution of methyl (E)-3-[2-n-butyl-1-{(2-chlorophenyl)methyl}-1H-imidazol-5-yl]-2-(4-pyridyl)methyl-2-propenoate (3.1 g, 7.3 mmol) in ethanol (16 mL) was treated with 10% sodium hydroxide solution and the mixture was stirred for 18 hours at 25°C. The solution was concentrated in vacuum, water was added, the pH was adjusted to 6.5 and the resulting solid was filtered, washed with water and crystallized from methanol/ether to afford 0.48 g of (E)-3-[2-n-butyl-1-{(2-chlorophenyl)methyl}-1H-imidazol-5-yl]-2-(4-pyridyl)methyl-2-propenoic acid; mp 178-182°C (d).

### Examples 6-7

In Table II are listed other examples of alkenoic acids prepared by the methods described in Preparation 1 (i-iv). The starting materials and products are shown in Table II.

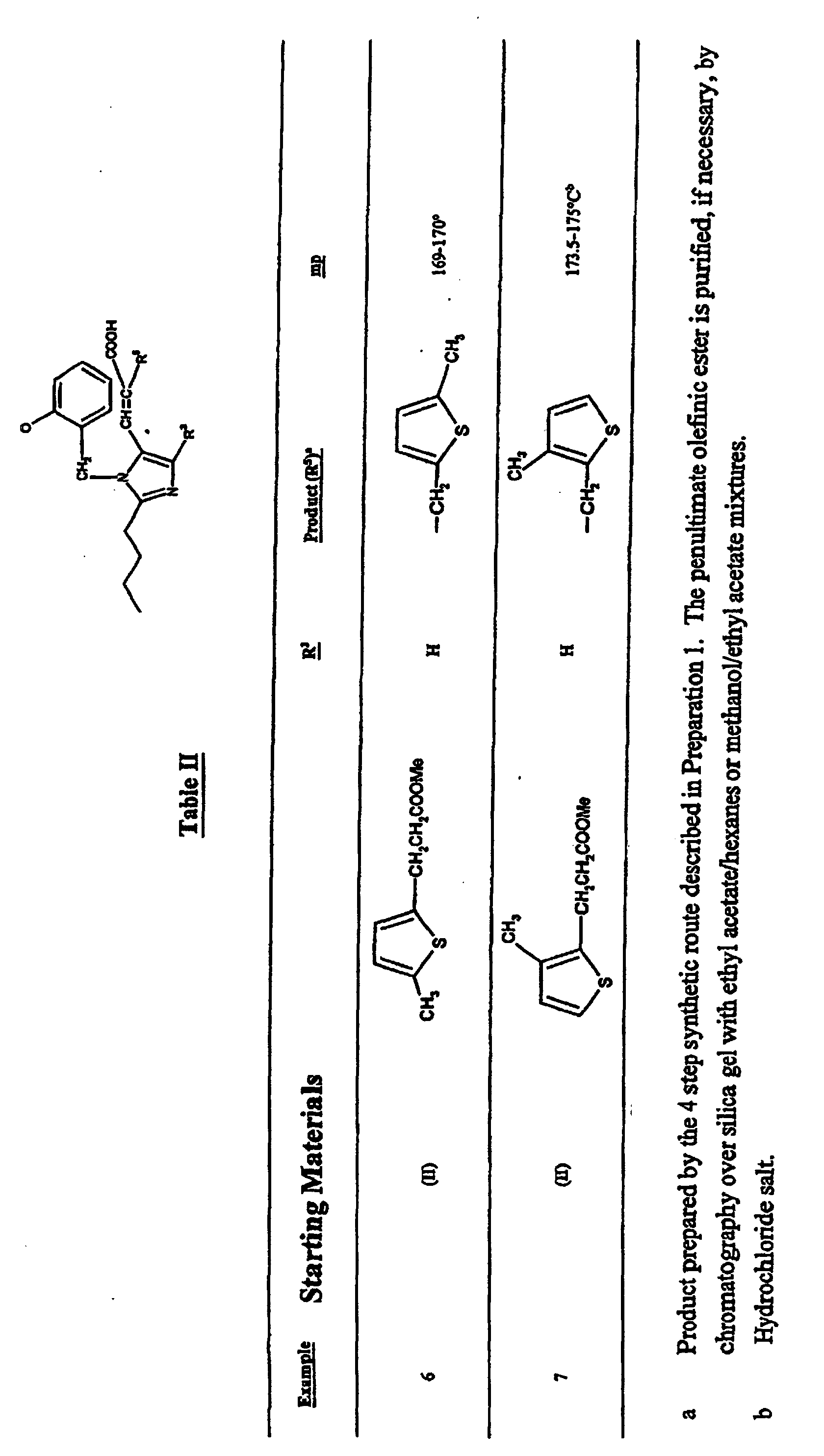

### Example 8

By the procedure of Example 1, using in place of 2-chlorobenzyl bromide, the following:
2-methylbenzyl bromide,
4-methoxybenzyl bromide, and
4-phenylbenzyl bromide;
and using the phosphonopropionate of Example 1, (MeO)₂P(O)CH(CH₂-2-thienyl)COOMe, the following products are obtained:
(E)-3-[2-n-butyl-1-{(2-methylphenyl)methyl}-1H-imidazol-5-yl]-2-(2-thienyl)methyl-2-propenoic acid,
(E)-3-[2-n-butyl-1-{(4-methoxyphenyl)methyl}-1H-imidazol-5-yl]-2-(2-thienyl)methyl-2-propenoic acid.

### Example 9

The following methyl ester of a propenoate are prepared as in Example 8:
methyl (E)-3-[2-n-butyl-1-[(4-methoxyphenyl)-methyl]-1H-imidazol-5-yl]-2-(2-thienyl)methyl-2-propenoate.

This is treated with boron tribromide in methylene chloride at room temperature for six hours and then the reaction mixture is condensed and treated with a mixture of ethyl acetate and water. The washed ethyl acetate layer gives on evaporation:
(E)-3-[2-n-butyl-1-[(4-hydroxyphenyl)methyl]-1H-imidazol-5-yl]-2-(2-thienyl)methyl-2-propenoic acid.

### Preparation 2

### (E)-3-[2-Phenyl-1-{(2-chlorophenyl)methyl}-1H-imidazol-5-yl]-2-(2-thienyl)methyl-2-propenoic Acid

By the procedure of Example 1(ii) Method 2, using benzamidine methyl ether in place of valeramidine methyl ether, 2-phenyl-5-hydroxymethylimidazole is prepared and converted to 2-phenyl-1-(2-chlorophenyl)methyl-5-hydroxy-methyl-1H-imidazole. The 5-hydroxymethyl group is oxidized using manganese dioxide by the procedure of Example 1 (iii). The resulting 2-phenyl-1-(2-chlorophenyl)methyl-1H-imidazol-5-carboxaldehyde is used in the procedure of Example 20 with methyl 3-(2-thienyl)propanoate to give the title compound.

### Example 10

Prepared by the procedure of Preparation 2 using the following amidine methyl ether:

C₂H₅C=NH(OCH₃);

the following product is obtained:
(E)-3-[2-ethyl-1-{(2-chlorophenyl)methyl}-1H-imidazol-5-yl]-2-(2-thienyl)methyl-2-propenoic acid.

### Example 11

### (E)-3-[2-n-Butyl-1-{(4-carboxyphenyl)methyl}-1H-imidazol-5-yl]-2-(2-thienyl)methyl-2-propenoic Acid

(i) By the procedure of Example 1 [(ii) Method 2, (iii) and (iv) Method B] using 4-carbomethoxybenzyl alcohol in place of 2-chlorobenzyl alcohol, the title compound was prepared; mp 250-253°C.

### Example 12

### (E)-3-[2-n-Butyl-1-{(4-carboxy-2-chlorophenyl)methyl}-1H-imidazol-5-yl]-2-(2-thienyl)methyl-2-propenoic Acid

A suspension of 2-butylimidazol-5-aldehyde (16,92 g, 0.111 mol, prepared by manganese dioxide oxidation of the alcohol, prepared in Example 1, Method 2), chloromethyl pivalate (21.77 g, 0.145 mol), and potassium carbonate (20.07 g, 0.145 mol) in 200 ml of dimethylformamide was stirred at ambient temperature under argon for four days. The solids were removed by filtration and washed with ether. The combined filtrates were partitioned between diethyl ether and water. The ether phase was washed successively with water and brine, dried over magnesium sulfite and concentrated under vacuum to give 23.6 g of 2-n-butyl-1-pivalyloxymethylimidazole-5-aldehyde.

A mixture of ethyl 4-bromomethyl-3-chlorobenzoate (5.28 g, 0.020 mol, U.S. Patent No. 4,837,333) and 2-n-butyl-1-pivaloyloxymethyl-imidazole-5-aldehyde (4.45 g, 0.0167 mol) was heated at 100°C under argon for 18 hours. Repeated trituration with ether gave 6.38 g of a crystalline salt. A suspension of this salt in 100 ml of ethyl acetate was stirred for 0.5 hours with 100 ml of 5% aqueous sodium carbonate. The layers were separated, the aqueous layer washed with ethyl acetate, and the combined organic layers washed with water, dried over magnesium sulfate and concentrated to give an oil. Chromatography of this oil over silica eluting gel with ethyl acetate/hexane (1:1) gave 1.02 g of 2-n-butyl-1-[(4-carboethoxy-2-chlorophenyl)-methyl]imidazole-5-aldehyde.

Ethyl 2-carboxy-3-(2-thienyl)propionate (14 g, 0.061 mol) was prepared by stirring a solution of diethyl 2-thienylmalonate (16.8 g, 0.0655 mol) and potassium hydroxide (4.41 g, 0.0786 mol) in 200 ml of ethanol under argon at room temperature for 12 days and then purifying by removing the solvent under vacuum, dissolving the reside in water, washing the aqueous layer with aqueous hydrochloric acid and with diethyl ether.

A solution of this half-acid, half-ester (1.05 g, 4.62 mmol) in 5 ml of toluene was added to a refluxing solution of 2-n-butyl-1-[(4-carboethoxy-2-chlorophenyl)methyl]-imidazole-5-aldehyde (1.03g, 3.08 mmol) and piperidine (0.26 g, 3.08 mmol) in 60 ml of toluene. Twice, at 1 hour intervals, an additional 1 g of the half-acid, half-ester was added, and the solution was then refluxed for 17 hours. Evaporation of the toluene and chromatography of the residue over silica gel using 2:3 ethyl acetate-hexane for elution gave 0.39 g of the diester of the title product. This was hydrolyzed in 2:1 ethanol-water with 5 equivalents of potassium hydroxide for 18 hours and worked up in the usual manner to give 0.260 g of final product; mp 234-236°C. The NMR of this product was in accord with its structure.

### Example 13

### (E)-3-[2-n-Butyl-1-{(4-sulfonamidophenyl)methyl}-1H-imidazol-5-yl]-2-(2-thienyl)methyl-2-propenoic Acid

The procedure of Example 12 is followed using 4-bromomethylbenzenesulfonamide (Braselton, et al., Anal. Chem., 48, 1386 (1976)) in place of methyl 4-bromomethyl-3-chlorobenzoate to give the title compound,

### Example 14

### (E)-3-[2-n-Butyl-1-{(4-carboxy-2-nitrophenyl)methyl}-1H-imidazol-5-yl]-2-(2-thienyl)methyl-2-propenoic Acid

The procedure of Example 12 was followed using methyl 4-bromomethyl-3-nitrobenzoate (prepared from 4-methyl-3-nitrobenzoic acid by esterification with gaseous hydrochloric acid-methanol followed by methyl bromination with N-bromosuccinimide) to give the title compound.

### Example 15

### (E)-3-[2-n-Butyl-1-{(4-carboxy-3-chlorophenyl)methyl}-1H-imidazol-5-yl]-2-(2-thienyl)methyl-2-propenoic Acid

The procedure of Example 12 was followed using ethyl 4-bromomethyl-2-chlorobenzoate (U.S. Patent No. 4,837,333) in place of ethyl 4-bromomethyl-3-chlorobenzoate to give the title compound; 245-246°C.

### Example 16

### (E)-3-[2-n-Butyl-1-{[2-chloro-4-(1H-tetrazol-5-yl)phenyl]methyl}-1H-imidazol-5-yl]-2-(2-thienyl)methyl-2 -propenoic Acid

The procedure of Example 12 is followed using t-butyl 4-bromomethyl-3-chlorobenzoate (prepared from 3-chloro-4-methylbenzoic acid by esterification with 2-methylpropene in the presence of concentrated sulfuric acid, followed by methyl bromination with N-bromosuccinimide) in place of ethyl 4-bromomethyl-3-chlorobenzoate to give ethyl (E)-3-[2-n-butyl-1-{[2-chloro-4-(carbo-t-butoxy)phenyl]methyl}-1H-imidazol-5-yl]-2-(2-thienyl)methyl-2-propenoate. The t-butyl ester is converted to the corresponding acid compound using trifluoroacetic acid.

To a suspension of ethyl (E)-3-[2-n-butyl-1-{(2-chloro-4-carboxyphenyl)methyl}-1H-imidazol-5-yl]-2-(2-thienyl)-methyl-2-propenoate in benzene is added thionyl chloride. The resultant mixture is heated to 50°C for 90 minutes, then evaporated to an oily residue. The residue is taken up in hexane and evaporated again. The acid chloride is treated with concentrated ammonium hydroxide and then the reaction mixture is stirred for 16 hours at room temperature. The solid is filtered, washed with water, and dried at 50°C under vacuum to yield the primary amide derivative.

To a solution of dimethylformamide in acetonitrile is added oxalyl chloride at 0°C under argon. After 3 minutes, a solution of the amide prepared above in dimethylformamide is added via a cannula. Five minutes later, pyridine is added; the reaction mixture is stirred for an additional 5 minutes at 0°C, then partitioned between ethyl acetate and 50% aqueous ammonium chloride. The ethyl acetate layer is washed with water and brine. The ethyl acetate extract is dried with anhydrous sodium sulfate and evaporated to give the corresponding nitrile derivative.

Tetrahydrofuran is added under argon with stirring to a mixture of the nitrile prepared above and aluminum chloride. Sodium azide is added all at once, followed by a tetrahydrofuran rinse, and the reaction is heated to 65°C for 22 hours, then cooled to room temperature. The reaction mixture is diluted with ethyl acetate and treated with 10% hydrochloric acid solution with vigorous stirring for 5 minutes. The ethyl acetate layer is washed with water and brine. The ethyl acetate layer is dried with anhydrous sodium sulfate and evaporated to give ethyl (E)-3-[2-n-butyl-1-{[2-chloro-4-(1H-tetrazol-5-yl)phenyl]methyl}-1H-imidazol-5-yl]-2-(2-thienyl)methyl-2-propenoate.

The title propenoic acid compound is prepared from the above ethyl ester by basic hydrolysis using aqueous base in methanol.

### Example 17

### (E)-[2-n-Butyl-1-{(2-nitrophenyl)methyl}-1H-imidazol-5-yl]-2-(2-thienyl)methyl-2-propenoic Acid

The title compound was prepared following the procedure of Example 1 using 2-nitrobenzyl bromide in place of 2-chlorobenzyl bromide; mp 205-206°C.

### Example 18

### (E)-[2-n-Butyl-1-{(3-nitrophenyl)methyl}-1H-imidazol-5-yl]-2-(2-thienyl)methyl-2-propenoic Acid

The title compound was prepared following the procedure of Example 1 using 3-nitrobenzyl alcohol in place of 2-chlorobenzyl alcohol; mp 182-184°C.

### Example 19

### (E)-[2-n-Butyl-1-{(4-nitrophenyl)methyl}-1H-imidazol-5-yl]-2-(2-thienyl)methyl-2-propenoic Acid

The title compound was prepared following the procedure of Example 12 using 4-nitrobenzyl bromide in place of ethyl-4-bromomethyl-3-chlorobenzoate; mp 198-200°C.

### Example 20

### (E)-[2-n-Butyl-1-{(2-trifluoromethylphenyl)methyl}-1H-imidazol-5-yl]-2-(2-thienyl)methyl-2-propenoic Acid

The title compound was prepared following the procedure of Example 1 using 2-trifluoromethylbenzyl alcohol in place of 2-chlorobenzyl alcohol; mp 202-203°C.

### Example 21

### (E)-[2-n-Butyl-1-{(2,3-dichlorophenyl)methyl}-1H-imidazol-5-yl]-2-(2-thienyl)methyl-2-propenoic Acid

The title compound was prepared following the procedure of Example 1 using 2,3-dichlorobenzyl alcohol in place of 2-chlorobenzyl alcohol; mp 184-185°C.

### Example 22

### (E)-[2-n-Butyl-1-{(3-methoxy-2-nitrophenyl)methyl}-1H-imidazol-5-yl]-2-(2-thienyl)methyl-2-propenoic Acid

The title compound was prepared following the procedure of Example 12 using 3-methoxy-2-nitrobenzyl bromide in place of ethyl 4-bromomethyl-3-chlorobenzoate; mp 213-215°C.

### Example 23

### (E)-[2-n-Butyl-1-{(2-cyanophenyl)methyl}-1H-imidazol-5-yl]-2-(2-thienyl)methyl-2-propenoic Acid

The title compound was prepared following the procedure of Example 1 using 2-cyanobenzyl bromide in place of ethyl 4-bromomethyl-3-chlorobenzoate; mp 210-212°C.

### Example 24

### (E)-[2-n-Butyl-1-{(4-methoxy-3-methylphenyl)methyl}-1H-imidazol-5-yl]-2-(2-thienyl)methyl-2-propenoic Acid

The title compound was prepared following the procedure of Example 12 using 4-methoxy-3-methylbenzyl bromide in place of ethyl 4-bromomethyl-3-chlorobenzoate; mp 140-141°C.

### Example 25

### (E)-[2-n-Butyl-1-{(3-methoxyphenyl)methyl}-1H-imidazol-5-yl]-2-(2-thienyl)methyl-2-propenoic Acid

The title compound was prepared following the procedure of Example 1 using 3-methoxybenzyl alcohol in place of 2-chlorobenzyl alcohol; mp 170-171°C.

### Example 26

### (E)-[2-n-Butyl-1-{(2-methoxyphenyl)methyl}-1H-imidazol-5-yl]-2-(2-thienyl)methyl-2-propenoic Acid

The title compound was prepared following the procedure of Example 1 using 2-methoxybenzyl alcohol and methanesulfonic anhydride in place of 2-chlorobenzyl alcohol and triflic anhydride; mp 186-187°C.

### Example 27

### (E)-[2-n-Butyl-1-{(2-hydroxyphenyl)methyl}-1H-imidazol-5-yl]-2-(2-thienyl)methyl-2-propenoic Acid

The title compound was prepared from the 2-methoxy compound prepared in Example 26 using boron tribromide in methylene chloride; 181-183°C.

### Example 28

### (E)-[2-n-Butyl-1-{(2-chlorophenyl)methyl}-1H-imidazol-5-yl]-2-(5-methoxy-2-thienyl)methyl-2-propenoic Acid

The title compound was prepared by the procedure of Example 1 using 3-(5-methoxy-2-thienyl)-2-phosphonopropionate in place of 3-(2-thienyl)-2-phosphonopropionate; mp 184-185.5°C.

### Example 29

### (E)-[2-n-Butyl-1-{(2-chlorophenyl)methyl}-1H-imidazol-5-yl]-2-(4-methoxy-2-thienyl)methyl-2-propenoic Acid

The title compound was prepared by the procedure of Example 1 using 3-(4-methoxy-2-thienyl)-2-phosphonopropionate in place of 3-(2-thienyl)-2-phosphonopropionate; mp 170-171°C.

### Example 30

### (E)-3-[2-n-Hexyl-1-{(4-carboxyphenyl)methyl}-1H-imidazol-5-yl]-2-(2-thienyl)methyl-2-propenoic Acid

The title compound was prepared following the procedure of Example 1, using caproylamidine methyl ether hydrochloride in place of valeramidine methyl ether hydrochloride and using 4-carbomethoxybenzyl alcohol in place of 2-chlorobenzyl alcohol; mp 210-212°C.

### Example 31

### (E)-3-[2-n-Propyl-1-{(2-nitrophenyl)methyl}-1H-imidazol-5-yl]-2-(2-thienyl)methyl-2-propenoic Acid

The title compound was prepared following the procedure of Example 1 using butyramidine methyl ether hydrochloride in place of valeramidine methyl ether hydrochloride and 2-nitrobenzyl alcohol in place of 2-chlorobenzyl alcohol; mp 223°C.

### Example 32

### E-3-[2-n-Butyl-1-{(2-carboxyphenyl)methyl}-1H-imidazol-5-yl]-2-(2-thienyl)methyl-2-propenoic Acid

The title compound was prepared using the procedure of Example 12 replacing. ethyl 4-bromomethyl-3-chlorobenzoate with ethyl 2-bromomethylbenzoate; 201-202°C.

### Example 33

### E-3-[2-n-Butyl-1-{(3-carboxyphenyl)methyl}-1H-imidazol-5-yl]-2-(2-thienyl)methyl-2-propenoic Acid

The title compound was prepared by the procedure of Example 1 (iv, Method B) using 2-n-butyl-1-[(4-carbo-methoxyphenyl)methyl]imidazole-5-aldehyde (prepared by the method described for the preparation of 2-n-butyl-1-[(4-carboethoxy-2-chlorophenyl)methyl]-imidazole-5-aldehyde in Example 12) and methyl 3-(2-thienyl)propanoate; mp 243-244°C.

### Example 34

### (E)-3-[2-n-Butyl-1-{(4-hydroxy-3-methylphenyl)methyl}-1H-imidazol-5-yl]-2-(2-thienyl)methyl-2-propenoic Acid

The title compound was prepared by demethylation of (E)-2-n-butyl-1-{(4-methoxy-3-methylphenyl)methyl}-1H-imidazol-5-yl]-2-(2-thienyl)methyl-2-propenoic acid, prepared in Example 24, using boron tribromide in methylene chloride; mp 150-152°C.

### Example 35

### (E)-3-[2-n-Butyl-1-{(4-carbomethoxyphenyl)methyl}-1H-imidazol-5-yl]-2-(2-thienyl)methyl-2-propenoic Acid

The title compound was prepared using 2-n-butyl-1-[(4-carbomethoxyphenyl)methyl]imidazole-5-aldehyde (prepared by the method described for the preparation of 2-n-butyl-1-[(4-carboethoxy-2-chlorophenyl)methyl]imidazole-5-aldehyde in Example 12) and t-butyl 3-(2-thienyl)-propanoate by the procedure of Example 1 (iv, Method B), except, instead of basic hydrolysis, trifluoroacetic acid hydrolysis of the t-butyl ester was employed; mp 217-220°C.

### Example 36

### (E)-3-[2-n-Butyl-1-{(4-cyanophenyl)methyl}-1H-imidazol-5-yl]-2-(2-thienyl)methyl-2-propenoic Acid

The title compound was prepared using 2-n-butyl-[(4-cyanophenyl)methyl]imidazole-5-aldehyde (prepared by the method of Example 12 describing the preparation of 2-n-butyl-1-[(4-carboethoxy-2-chlorophenyl)methyl]imidazole-5-aldehyde) and methyl 3-(2-thienyl)propanoate by the procedure of Example 1 (iv, Method B), except, instead of basic hydrolysis of the ester with sodium hydroxide, potassium carbonate hydrolysis was employed; mp 190-192°C.

### Example 37

### (E)-3-[2-n-Butyl-1-{(4-carbamoylphenyl)methyl}-1H-imidazol-5-yl]-2-(2-thienyl)methyl-2-propenoic Acid

Methyl (E)-3-[2-n-butyl-1-{(4-cyanophenyl)methyl}-1H-imidazol-5-yl]-2-(2-thienyl)methyl-2-propanoate, prepared in Example 36, was subjected to hydrolysis with concentrated hydrochloric acid to give the title compound; mp 210-212°C.

### Example 38

### (E)-3-[2-n-Butyl-1-{[4-(1H-tetrazol-5-yl)-phenyl]methyl}-1H-imidazol-5-yl]-2-(2-thienyl)-methyl-2-propenoic Acid

The title compound was prepared from methyl (E)-3-[2-n-butyl-1-{(4-cyanophenyl)methyl}-1H-imidazol-5-yl]-2-(2-thienyl)methyl-2-propanoate, prepared in Example 36, using the procedure described in Example 16; mp 246-248°C.

### Example 39

### (E)-3-[2-n-Propyl-1-{(4-carboxyphenyl)methyl}-1H-imidazol-5-yl]-2-(2-thienyl)methyl-2-propenoic Acid

The title compound was prepared using the procedure of Example I replacing valeramidine methyl ether hydrochloride with butyramidine methyl ether hydrochloride and replacing 2-chlorobenzyl alcohol with 4-carbomethoxybenzyl alcohol; mp 250°C (d).

### Example 40

### (E)-3-[2-n-Propyl-1-{(2-chlorophenyl)methyl}-1H-imidazol-5-yl]-2-(2-thienyl)methyl-2-propenoic Acid

The title compound was prepared using the procedure of Example 1 replacing valeramidine methyl ether hydrochloride with butyramidine methyl ether hydrochloride; mp 200°C.

### Example 41

### (E)-3-[2-n-Hexyl-1-{(2-chlorophenyl)methyl}-1H-imidazol-5-yl]-2-(2-thienyl)methyl-2-propenoic Acid

The title compound was prepared using the procedure of Example 1 replacing valeramidine methyl ether hydrochloride with caproylamidine methyl ether hydrochloride; mp 161-163°C.

### Example 42

### (E)-3-[2-n-Butyl-1-{(4-carboxy-2,3-dichlorophenylmethyl}1H-imidazol-5-yl]-2-(2-thienyl)methyl-2-propenoic Acid

The title compound is prepared using the procedure of Example 12 replacing 4-bromomethyl-2,3-dichlorobenzoate (prepared by oxidation of 2,3-dichlorobenzoate (prepared by oxidation of 2,3-dichloro-p-xylene with nitric acid, followed by esterification with methanol/hydrochloric acid, and methyl bromination with N,N-bromosuccinimide).

### Example 43

### (E)-3-[2-n-Butyl-1-{(4-carboxy-2,5-dichlorophenyl)methyl}-1H-imidazol-5-yl]-2-(2-thienyl)methyl-2-propenoic Acid

The title compound was prepared using the procedure of Example 12 replacing ethyl 4-bromomethyl-3-chlorobenzoate with methyl 4-bromomethyl-3,6-dichlorobenzoate (prepared by oxidation of 2,5-dichloro-p-xylene with nitric acid, followed by esterification with methanol/hydrochloric acid, and methyl bromination with N-bromosuccinimide).

### Example 44

An oral dosage form for administering orally active Formula (I) compounds is produced by screening, mixing and filling into hard gelatin capsules the ingredients in proportions, for example, as shown below.

| Ingredients | Amounts |
|---|---|
| (E)-3-[2-n-butyl-1-{(4-carboxyphenyl)methyl}-1H-imidazol-5-yl]-2-(2-thienyl)methyl-2-propenoic acid | 100 mg |
| magnesium stearate | 10 mg |
| lactose | 100 mg |

### Example 45

The sucrose calcium sulfate dihydrate and orally active Formula (I) compounds are mixed and granulated with a 10% gelatin solution. The wet granules are screened, dried, mixed with the starch, talc and stearic acid, screened and compressed into a tablet.

| Ingredients | Amounts |
|---|---|
| (E)-3-[2-n-butyl-1-{(4-carboxy-2-chlorophenyl)-methyl}-1H-imidazol-5-yl]-2-(2-thienyl)methyl-2-propenoic acid | 75 mg |
| calcium sulfate dihydrate | 100 mg |
| sucrose | 15 mg |
| starch | 8 mg |
| talc | 4 mg |
| stearic acid | 2 mg |

### Example 46

(E)-3-[2-n-Butyl-1-{(4-carboxy-3-chlorophenyl)methyl}-1H-imidazol-5-yl]-2-(2-thienyl)methyl-2-propenoic acid, 50 mg, is dispersed in 25 mL of normal saline to prepare an injectable preparation.

### Example 47

A topical opthamological solution for administering Formula (I) compounds is produced by mixing under sterile conditions the ingredients in proportions, for example, as shown below.

| Ingredients | Amounts |
|---|---|
| | (mg/mL) |
| (E)-3-[2-n-butyl-1-{2-chlorophenyl)methyl]-1H-imidazol-5-yl]-2-(2-thienyl)methyl-2-propenoic acid | 1.0 |
| dibasic sodium phosphate | 10.4 |
| monobasic sodium phosphate | 2.4 |
| chlorobutanol | 5.0 |
| hydroxypropanol methylcellulose | 5.0 |
| sterile water | q.s.ad 1.0mL |
| 1.0 N sodium hydroxide | q.s.ad pH 7.4 |

It is to be understood that the invention is not limited to the embodiments illustrated hereabove and the right to the illustrated embodiments and all modifications coming within the scope of the following claims is reserved.

## Claims (Claims for the following Contracting State(s): AT, BE, CH, DE, DK, FR, GB, IT, LI, LU, NL, SE)

1. A compound of the formula (I): 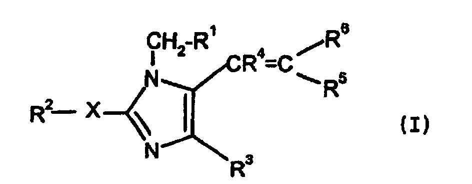 in which:
R¹ is phenyl unsubstituted or substituted by one to three substituents selected from chloro, fluoro, trifluoromethyl, nitro, methyl, methoxy, hydroxy, sulfamido, carboxy, carboC₁-C₄alkoxy, carbamoyl, cyano, or tetrazol-5-yl wherein n is 1-3;
R² is C₂-C₈alkyl;
X is a single bond;
R³ is hydrogen;
R⁴ is hydrogen;
R⁵ is thienylmethyl optionally substituted by methyl or methoxy, and
R⁶ is COOH;
or a pharmaceutically acceptable salt thereof, wherein the compound of formula (I) is the E (trans) isomer.

2. A compound according to claim 1 which is:
(E)-3-[2-n-butyl-1-{(4-carboxy-3-chlorophenyl)methyl}-1H-imidazol-5-yl]-2-(2-thienyl)methyl-2-propenoic acid;
(E)-3-[2-n-butyl-1-{(2-chlorophenyl)methyl}-1H-imidazol-5-yl]-2-(2-thienyl)methyl-2-propenoic acid;
(E)-3-[2-n-butyl-1-{(4-carboxy-2-chlorophenyl)methyl}-1H-imidazol-5-yl]-2-(2-thienyl)methyl-2-propenoic acid;
(E)-3-[2-n-butyl-1-{(4-carbomethoxyphenyl)methyl}-1H-imidazol-5-yl]-2-(2-thienyl)methyl-2-propenoic acid;
(E)-3-[2-n-hexyl-1-{(4-carboxyphenyl)methyl}-1H-imidazol-5-yl]-2-(2-thienyl)methyl-2-propenoic acid;
(E)-3-[2-n-propyl-1-{(4-carboxyphenyl)methyl}-1H-imidazol-5-yl]-2-(2-thienyl)methyl-2-propenoic acid;
(E)-3-[2-n-butyl-1-{(2-nitrophenyl)methyl}-1H-imidazol-5-yl]-2-(2-thienyl)methyl-2-propenoic acid;
(E)-3-[2-n-butyl-1-{(2-chlorophenyl)methyl}-1H-imidazol-5-yl]-2-(3-thienyl)methyl-2-propenoic acid;
(E)-3-[2-n-butyl-1-{(2-chlorophenyl)methyl}-1H-imidazol-5-yl]-2-(5-methyl-2-thienyl)methyl-2-propenoic acid;
(E)-3-[2-n-butyl-1-{(2-cyanophenyl)methyl}-1H-imidazol-5-yl]-2-(2-thienyl)methyl-2-propenoic acid;
(E)-3-[2-n-butyl-1-{(4-methoxy-3-methylphenyl)methyl}-1H-imidazol-5-yl]-2-(2-thienyl)methyl-2-propenoic acid;
(E)-3-[2-n-butyl-1-{(2-chlorophenyl)methyl}-1H-imidazol-5-yl]-2-(5-methoxy-2-thienyl)methyl-2-propenoic acid;
(E)-3-[2-n-butyl-1-{(2,3-dichlorophenyl)methyl}-1H-imidazol-5-yl]-2-(2-thienyl)methyl-2-propenoic acid; or
(E)-3-[2-n-butyl-1-{(2-trifluoromethylphenyl)methyl}-1H-imidazol-5-yl]-2-(2-thienyl)methyl-2-propenoic acid; or a pharmaceutically acceptable salt thereof.

3. A compound according to claim 1 or 2 for use as a medicament.

4. A pharmaceutical composition which comprises a compound according to either claim 1 or claim 2 and a pharmaceutically acceptable carrier.

5. A process for preparing a compound of the formula (I) or a pharmaceutically acceptable salt thereof as defined in claim 1 or claim 2, which process comprises:
a) reacting a compound of the formula (II): 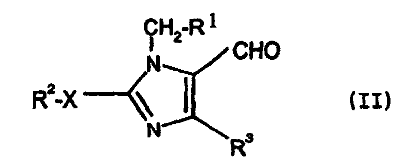 wherein R², R³, and X are as defined in claim 1, and R¹ as defined in claim 1, except that the substituents on the R¹ group do not include tetrazol-5-yl, OH, or CO₂H, with (C₁-C₄alkoxy)₂P(O)CH(R⁵)-COOC₁-C₆alkyl, wherein R⁵ is as defined in claim 1, in the presence of a base; or
b) reacting a compound of the formula (III): 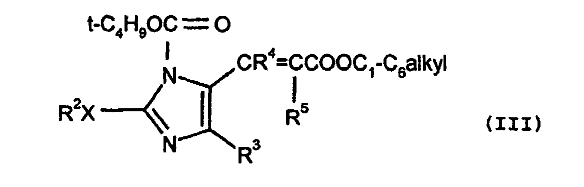 wherein R², R³, R⁴, R⁵, and X are as defined in claim 1, with a compound of the formula (IV): 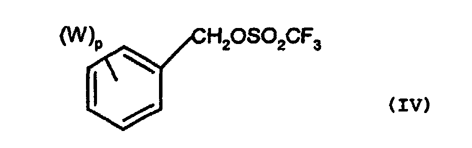 wherein W is Cl, Br, F, I, C₁-C₄alkyl, nitro, CO₂C₁-C₄alkyl, C₁-C₄alkoxy, SC₁-C₄alkyl, CN, SO₂C₁-C₄alkyl, SO₂NHR⁷, NHSO₂C₁-C₄alkyl, or CₙF₂ₙ₊₁, n is 1-3, p is 0-3, and R⁷ is hydrogen, C₁₋₄alkyl, or (CH₂)₀₋₄phenyl; or
c) reacting a compound of the formula (II) as hereinbefore defined with a compound of the formula (V): 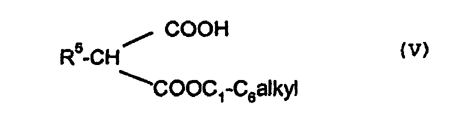 wherein R⁵ is as defined in claim 1, in the presence of a base; or
d) reacting a compound of the formula (VI): 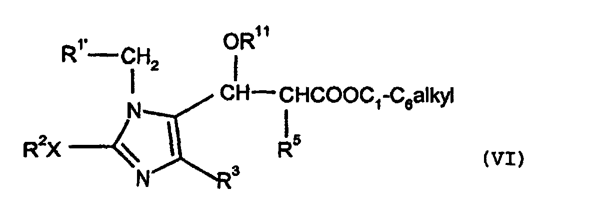 wherein R², R³, R⁵, and X are as defined in claim 1, R^{1'} is R¹ as defined in claim 1, except that the substituents on the R^{1'} group do not include tetrazol-5-yl, OH, or CO₂H and R¹¹ is COCH₃ or SO₂CH₃, with a base; or
e) reacting a compound of the formula (II) as hereinbefore defined with an appropriate heterocyclic acetic acid in acetic anhydride in the presence of a base;
and thereafter where necessary:
(i) for formula (I) compounds in which the R¹ group is substituted by hydroxy, deprotecting the formula (I) compounds in which the R¹ group is substituted by C₁-C₄alkoxy; or
(ii) for formula (I) compounds in which the R¹ group is substituted by carboxy, hydrolyzing the formula (I) compounds in which the R¹ group is substituted by CO₂C₁₋₄alkyl; or
(iii) for formula (I) compounds in which R¹ group is substituted by a tetrazol-5-yl group, treating the formula (I) compound in which the R¹ group is substituted by carboxy, with a halogenating agent, followed by conversion to the primary amide in a reaction with ammonia, dehydration with oxalylchloride/dimethylformamide and reaction with azide; or
(iv) for formula (I) compounds in which R⁶ is -COOH, hydrolyzing the formula (I) compounds in which R⁶ is -COOC₁-C₆alkyl;
and thereafter optionally forming a pharmaceutically acceptable salt.

6. The use of a compound of the formula (I) or a pharmaceutically acceptable salt thereof as defined in either claim 1 or claim 2 in the manufacture of a medicament for treatment of diseases in which angiotensin II receptor antagonism is a factor.

7. The use of a compound of the formula (I) or a pharmaceutically acceptable salt thereof as defined in either claim 1 or claim 2 in the manufacture of a medicament for the treatment of hypertension, congestive heart failure or renal failure.

## Claims (Claims for the following Contracting State(s): ES, GR)

1. A process for preparing a compound of the formula (I): 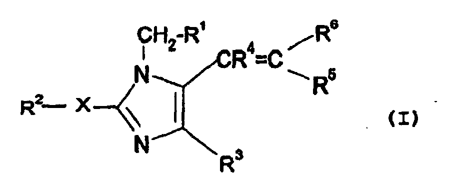 in which:
R¹ is phenyl unsubstituted or substituted by one to three substituents selected from chloro, fluoro, trifluoromethyl, nitro, methyl, methoxy, hydroxy, sulfamido, carboxy, carboC₁-C₄alkoxy, carbamoyl, cyano, or tetrazol-5-yl;
X is a single bond;
R² is C₂-C₈alkyl;
R³ is hydrogen;
R⁴ is hydrogen;
R⁵ is thienylmethyl optionally substituted by methyl or methoxy; and
R⁶ is COOH wherein the compound of formula (I) is the E (trans) isomer; or a pharmaceutically acceptable salt thereof;
which process comprises:
a) reacting a compound of the formula (II): 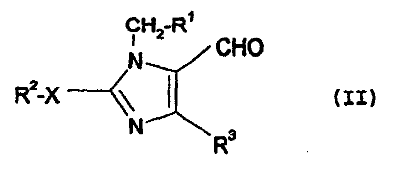 wherein R², R³, and X are as defined in claim 1, and R¹ as defined in claim I, except that the substituents on the R¹ group do not include tetrazol-5-yl, OH, or CO₂H, with (C₁-C₄alkoxy)₂P(O)CH(R⁵)-COOC₁-C₆alkyl, wherein R⁵ is as defined in claim 1, in the presence of a base; or
b) reacting a compound of the formula (III): 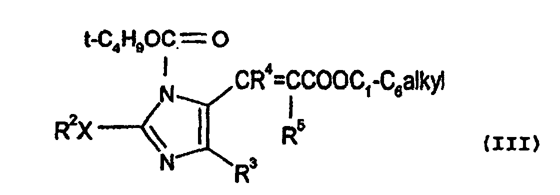 wherein R², R³, R⁴, R⁵, and X are as defined in claim 1, with a compound of the formula (IV): 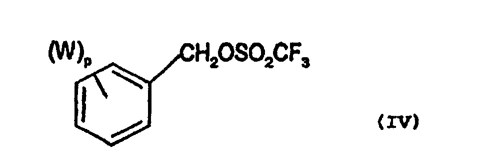 wherein W is Cl, Br, F, I, C₁-C₄alkyl, nitro, CO₂C₁-C₄alkyl, C₁-C₄alkoxy, SC₁-C₄alkyl, CN, SO₂C₁-C₄alkyl, SO₂NHR⁷, NHSO₂C₁-C₄alkyl, or CₙF₂ₙ₊₁, n is 1-3, p is 0-3, and R⁷ is hydrogen, C₁₋₄alkyl, or (CH₂)₀₋₄phenyl; or
c) reacting a compound of the formula (II) as hereinbefore defined with a compound of the formula (V): 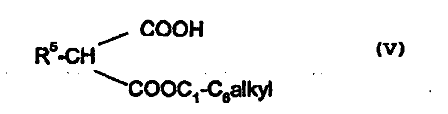 wherein R⁵ is as defined in claim 1, in the presence of a base; or
d) reacting a compound of the formula (VI): 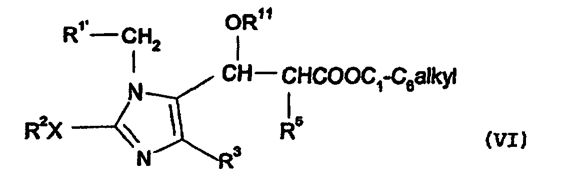 wherein R², R³, R⁵, and X are as defined in claim 1, R^{1'} is R¹ as defined in claim 1, except that the substituents on the R^{1'} goup do not include tetrazol-5-yl, OH, or CO₂H and R¹¹ is COCH₃ or SO₂CH₃, with a base; or
e) reacting a compound of the formula (II) as hereinbefore defined with an appropriate heterocyclic acetic acid in acetic anhydride in the presence of a base;
and thereafter where necessary:
(i) for formula (I) compounds in which the R¹ group is substituted by hydroxy, deprotecting the formula (I) compounds in which the R¹ group is substituted by C₁-C₄alkoxy; or
(ii) for formula (I) compounds in which the R¹ group is substituted by carboxy, hydrolyzing the formula (I) compounds in which the R¹ group is substituted by CO₂C₁₋₄alkyl; or
(iii) for formula (I) compounds in which R¹ group is substituted by a tetrazol-5-yl group, treating the formula (I) compound in which the R¹ group is substituted by carboxy, with a halogenating agent, followed by conversion to the primary amide in a reaction with ammonia, dehydration with oxalylchloride/dimethylformamide and reaction with azide; or
(iv) for formula (I) compounds in which R⁶ is -COON, hydrolyzing the formula (I) compounds in which R⁶ is -COOC₁-C₆alkyl;
and thereafter optionally forming a pharmaceutically acceptable salt.

2. A process according to claim 1 wherein the compound of formula (I) is:
(E)-3-[2-n-butyl-1-{(4-carboxy-3-chlorophenyl)methyl}-1H-imidazol-5-yl]-2-(2-thienyl)methyl-2-propenoic acid;
(E)-3-[2-n-butyl-1-{(2-chlorophenyl)methyl}-1H-imidazol-5-yl]-2-(2-thienyl)methyl-2-propenoic acid;
(E)-3-[2-n-butyl-1-{(4-carboxy-2-chlorophenyl)methyl}-1H-imidazol-5-yl]-2-(2-thienyl)methyl-2-propenoic acid;
(E)-3-[2-n-butyl-1-{(4-carbomethoxyphenyl)methyl}-1H-imidazol-5-yl]-2-(2-thienyl)methyl-2-propenoic acid;
(E)-3-[2-n-hexyl-1-{(4-carboxyphenyl)methyl}-1H-imidazol-5-yl]-2-(2-thienyl)methyl-2-propenoic acid;
(E)-3-[2-n-propyl-1-{(4-carboxyphenyl)methyl}-1H-imidazol-5-yl]-2-(2-thienyl)methyl-2-propenoic acid;
(E)-3-[2-n-butyl-1-{(2-nitrophenyl)methyl}-1H-imidazol-5-yl]-2-(2-thienyl)methyl-2-propenoic acid;
(E)-3-[2-n-butyl-1-{(2-chlorophenyl)methyl}-1H-imidazol-5-yl]-2-(3-thienyl)methyl-2-propenoic acid;
(E)-3-[2-n-butyl-1-{(2-chlorophenyl)methyl}-1H-imidazol-5-yl]-2-(5-methyl-2-thienyl)methyl-2-propenoic acid;
(E)-3-[2-n-butyl-1-{(2-cyanophenyl)methyl}-1H-imidazol-5-yl]-2-(2-thienyl)methyl-2-propenoic acid;
(E)-3-[2-n-butyl-1-{(4-methoxy-3-methylphenyl)methyl}-1H-imidazol-5-yl]-2-(2-thienyl)methyl-2-propenoic acid;
(E)-3-[2-n-butyl-1-{(2-chlorophenyl)methyl}-1H-imidazol-5-yl]-2-(5-methoxy-2-thienyl)methyl-2-propenoic acid;
(E)-3-[2-n-butyl-1-{(2,3-dichlorophenyl)methyl}-1H-imidazol-5-yl]-2-(2-thienyl)methyl-2-propenoic acid; or
(E)-3-[2-n-butyl-1-{(2-trifluoromethylphenyl)methyl}-1H-imidazol-5-yl]-2-(2-thienyl)methyl-2-propenoic acid; or a pharmaceutically acceptable salt thereof.

3. A compound of the formula (I): 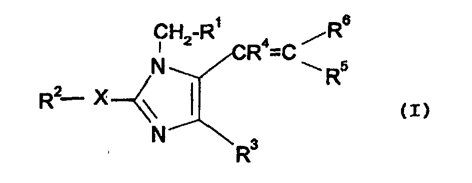 in which:
R¹ is phenyl unsubstituted or substituted by one to three substituents selected from chloro, fluoro, trifluoromethyl, nitro, methyl, methoxy, hydroxy, sulfamido, carboxy, carboC₁-C₄alkoxy, carbamoyl, cyano, or tetrazol-5-yl;
X is a single bond;
R² is C₂-C₈alkyl;
R³ is hydrogen;
R⁴ is hydrogen;
R⁵ is thienylmethyl optionally substituted by methyl or methoxy; and
R⁶ is COOH wherein the compound of formula (I) is the E (trans) isomer; or a pharmaceutically acceptable salt thereof.

4. A compound according to claim 3 for use as a medicament.

5. A pharmaceutical composition which comprises a compound according to claim 3 and a pharmaceutically acceptable carrier.

6. The use of a compound of the formula (I) or a pharmaceutically acceptable salt thereof as defined in claim 3 in the manufacture of a medicament for treatment of diseases in which angiotensin II receptor antagonism is a factor.

7. The use of a compound of the formula (I) or a pharmaceutically acceptable salt thereof as defined in claim 3 in the manufacture of a medicament for the treatment of hypertension, congestive heart failure or renal failure.

## Patentansprüche (Patentansprüche für folgende(n) Vertragsstaat(en): AT, BE, CH, DE, DK, FR, GB, IT, LI, LU, NL, SE)

1. Verbindung der Formel (I): 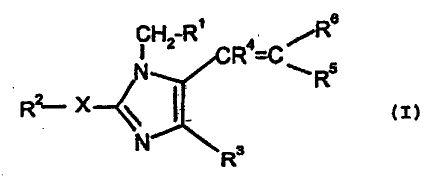 in der
R¹ eine Phenylgruppe ist, welche unsubstituiert oder mit einem bis drei Substituenten substituiert ist, welche ausgewählt sind aus einem Chloratom, Fluoratom, einer Trifluormethyl-, Nitro-, Methyl-, Methoxy-, Hydroxygruppe, einem Sulfamido-, Carboxy-, Carbo-C₁₋₄-alkoxy-, Carbamoyl-, Cyano- oder Tetrazol-5-ylrest;
R² für einen C₂-C₈-Alkylrest steht;
X eine Einfachbindung ist;
R³ ein Wasserstoffatom ist;
R⁴ ein Wasserstoffatom ist;
R⁵ für eine Thienylmethylgruppe, die gegebenenfalls mit einer Methyl- oder Methoxygruppe substituiert ist, steht; und
R⁶ für COOH steht;
oder ein pharmazeutisch verträgliches Salz davon, wobei die Verbindung der Formel (I) das E(trans)-Isomer ist.

2. Verbindung gemäß Anspruch 1, welche darstellt:
(E)-3-[2-n-Butyl-1-{(4-carboxy-3-chlorphenyl)methyl}-1H-imidazol-5-yl]-2-(2-thienyl)methyl-2-propensäure;
(E)-3-[2-n-Butyl-1-{(2-chlorphenyl)methyl}-1H-imidazol-5-yl]-2-(2-thienyl)methyl-2-propensäure;
(E)-3-[2-n-Butyl-1-{(4-carboxy-2-chlorphenyl)methyl}-1H-imidazol-5-yl]-2-(2-thienyl)methyl-2-propensäure;
(E)-3-[2-n-Butyl-1-{(4-carbomethoxyphenyl)methyl}-1H-imidazol-5-yl]-2-(2-thienyl)methyl-2-propensäure;
(E)-3-[2-n-Hexyl-1-{(4-carboxyphenyl)methyl}-1H-imidazol-5-yl]-2-(2-thienyl)methyl-2-propensäure;
(E)-3-[2-n-Propyl-1-{(4-carboxyphenyl)methyl}-1H-imidazol-5-yl]-2-(2-thienyl)methyl-2-propensäure;
(E)-3-[2-n-Butyl-1-{(2-nitrophenyl)methyl}-1H-imidazol-5-yl]-2-(2-thienyl)methyl-2-propensäure;
(E)-3-[2-n-Butyl-1-{(2-chlorphenyl)methyl}-1H-imidazol-5-yl]-2-(3-thienyl)methyl-2-propensäure;
(E)-3-[2-n-Butyl-1-{(2-chlorphenyl)methyl}-1H-imidazol-5-yl]-2-(5-methyl-2-thienyl)methyl-2-propensäure;
(E)-3-[2-n-Butyl-1-{(2-cyanophenyl)methyl}-1H-imidazol-5-yl]-2-(2-thienyl)methyl-2-propensäure;
(E)-3-[2-n-Butyl-1-{(4-methoxy-3-methylphenyl)methyl}-1H-imidazol-5-yl]-2-(2-thienyl)methyl-2-propensäure;
(E)-3-[2-n-Butyl-1-{(2-chlorphenyl)methyl}-1H-imidazol-5-yl]-2-(5-methoxy-2-thienyl)methyl-2-propensäure;
(E)-3-[2-n-Butyl-1-{(2,3-dichlorphenyl)methyl}-1H-imidazol-5-yl]-2-(2-thienyl)methyl-2-propensäure; oder
(E)-3-[2-n-Butyl-1-{(2-trifluormethylphenyl)methyl}-1H-imidazol-5-yl]-2-(2-thienyl)methyl-2-propensäure; oder ein pharmazeutisch verträgliches Salz davon.

3. Verbindung gemäß Anspruch 1 oder 2 zur Verwendung als Medikament.

4. Arzneimittel, welches eine Verbindung entweder gemäß Anspruch 1 oder Anspruch 2 und einen pharmazeutisch verträglichen Träger umfasst.

5. Verfahren zur Herstellung einer Verbindung der Formel (I) oder eines pharmazeutisch verträglichen Salzes davon wie in Anspruch 1 oder Anspruch 2 definiert, wobei das Verfahren umfasst:
a) Umsetzen einer Verbindung der Formel (II): 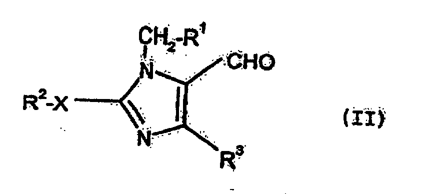 wobei R², R³ und X wie in Anspruch 1 definiert sind und R¹ wie in Anspruch 1 definiert ist, mit der Ausnahme, dass die Substituenten am Rest R¹ keine Tetrazol-5-ylgruppe, kein OH oder CO₂H einschließen, mit (C₁-C₄-Alkoxy)₂P(O)CH(R⁵)-COOC₁-C₆-alkyl, wobei R⁵ wie in Anspruch 1 definiert ist, in Gegenwart einer Base; oder
b) Umsetzen einer Verbindung der Formel (III): 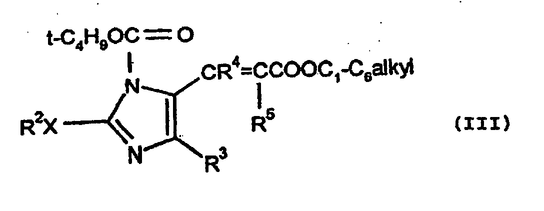 wobei R², R³, R⁴, R⁵ und X wie in Anspruch 1 definiert sind, mit einer Verbindung der Formel (IV): 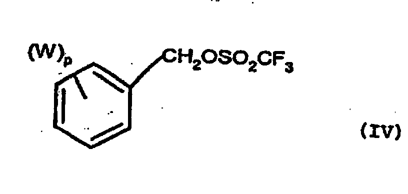 wobei W für Cl, Br, F, I, einen C₁-C₄-Alkylrest, eine Nitrogruppe, einen CO₂C₁-C₄-Alkylrest, einen C₁-C₄-Alkoxyrest, einen SC₁-C₄-Alkylrest, CN, einen SO₂C₁-C₄-Alkylrest, SO₂NHR⁷, einen NHSO₂C₁-C₄-Alkylrest oder CₙF₂ₙ₊₁ steht, n für 1 bis 3 steht, p für 0 bis 3 steht und R⁷ ein Wasserstoffatom, ein C₁-₄-Alkylrest oder (CH₂)₀₋₄-Phenylrest ist; oder
c) Umsetzen einer Verbindung der Formel (II) wie vorstehend definiert mit einer Verbindung der Formel (V): 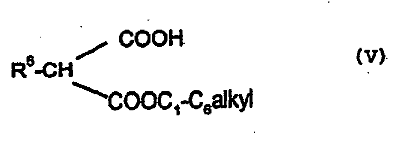 wobei R⁵ wie in Anspruch 1 definiert ist, in Gegenwart einer Base; oder
d) Umsetzen einer Verbindung der Formel (VI): 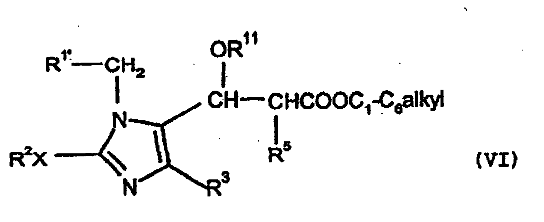 wobei R², R³, R⁵ und X wie in Anspruch 1 definiert sind, R^{1'} für R¹, wie in Anspruch 1 definiert, steht, mit der Ausnahme, dass die Substituenten am Rest R^{1'} keine Tetrazol-5-ylgruppe, kein OH oder CO₂H einschließen und R¹¹ für COCH₃ oder SO₂CH₃ steht, mit einer Base; oder
e) Umsetzen einer Verbindung der Formel (II) wie vorstehend definiert mit einer geeigneten heterocyclischen Essigsäure in Acetanhydrid in Gegenwart einer Base;
und anschließend, wenn nötig:
(i) bei Verbindungen der Formel (I), in denen der Rest R¹ mit einer Hydroxygruppe substituiert ist, Entfernen der Schutzgruppen der Verbindungen der Formel (I), in denen der Rest R¹ mit einem C₁-C₄-Alkoxyrest substituiert ist; oder
(ii) bei Verbindungen der Formel (I), in denen der Rest R¹ mit einer Carboxygruppe substituiert ist, Hydrolysieren der Verbindungen der Formel (I), in denen der Rest R¹ mit einem CO₂C₁₋₄-Alkylrest substituiert ist; oder
(iii) bei Verbindungen der Formel (I), in denen der Rest R¹ mit einer Tetrazol-5-ylgruppe substituiert ist, Behandeln der Verbindung der Formel (I), in der der Rest R¹ mit einer Carboxygruppe substituiert ist, mit einem Halogenierungsmittel, anschließend Umwandeln in das primäre Amid durch eine Umsetzung mit Ammoniak, Dehydrieren mit Oxalylchlorid/Dimethylformamid und Umsetzen mit Azid; oder
(iv) bei Verbindungen der Formel (I), in denen R⁶ für -COOH steht, Hydrolysieren der Verbindungen der Formel (I), in denen R⁶ ein -COOC₁-C₆-Alkylrest ist;
und gegebenenfalls anschließendes Bilden eines pharmazeutisch verträglichen Salzes.

6. Verwendung einer Verbindung der Formel (I) oder eines pharmazeutisch verträglichen Salzes davon wie entweder in Anspruch 1 oder Anspruch 2 definiert, zur Herstellung eines Medikaments zur Behandlung von Erkrankungen, bei denen ein Faktor der Angiotensin II-Rezeptorantagonismus ist.

7. Verwendung einer Verbindung der Formel (I) oder eines pharmazeutisch verträglichen Salzes davon wie entweder in Anspruch 1 oder Anspruch 2 definiert, zur Herstellung eines Medikaments zur Behandlung von Hypertonie, dekompensierter Herzinsuffizienz oder Nierenversagen.

## Patentansprüche (Patentansprüche für folgende(n) Vertragsstaat(en): ES, GR)

1. Verfahren zur Herstellung einer Verbindung der Formel (I): 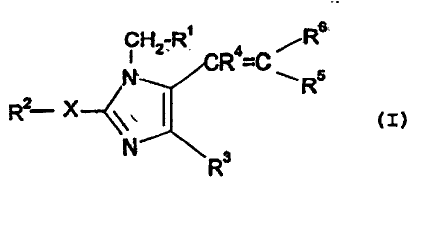 in der
R¹ eine Phenylgruppe ist, welche unsubstituiert oder mit einem bis drei Substituenten substituiert ist, welche ausgewählt sind aus einem Chloratom, Fluoratom, einer Trifluormethyl-, Nitro-, Methyl-, Methoxy-, Hydroxygruppe, einem Sulfamido-, Carboxy-, Carbo-C₁₋₄-alkoxy-, Carbamoyl-, Cyano- oder Tetrazol-5-ylrest;
X eine Einfachbindung ist;
R² für einen C₂-C₈-Alkylrest steht;
R³ ein Wasserstoffatom ist;
R⁴ ein Wasserstoffatom ist;
R⁵ für eine Thienylmethylgruppe, die gegebenenfalls mit einer Methyl- oder Methoxygruppe substituiert ist, steht; und
R⁶ für COOH steht, wobei die Verbindung der Formel (I) das E(trans)-Isomer ist;
oder eines pharmazeutisch verträglichen Salzes davon;
wobei das Verfahren umfasst:
a) Umsetzen einer Verbindung der Formel (II): 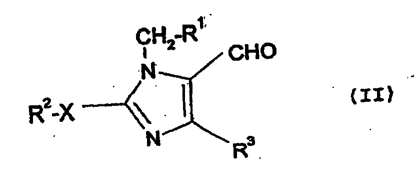 wobei R², R³ und X wie in Anspruch 1 definiert sind und R¹ wie in Anspruch 1 definiert ist, mit der Ausnahme, dass die Substituenten am Rest R¹ keine Tetrazol-5-ylgruppe, kein OH oder CO₂H einschließen, mit (C₁-C₄-Alkoxy)₂P(O)CH(R⁵)-COOCl-C₆-alkyl, wobei R⁵ wie in Anspruch 1 definiert ist, in Gegenwart einer Base; oder
b) Umsetzen einer Verbindung der Formel (III): 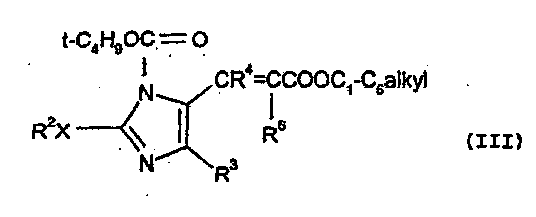 wobei R², R³, R⁴, R⁵ und X wie in Anspruch 1 definiert sind, mit einer Verbindung der Formel (IV): 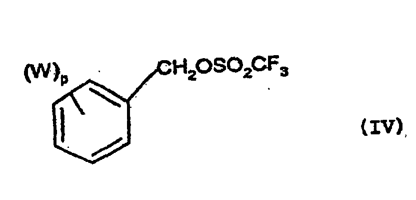 wobei W für Cl, Br, F, I, einen C₁₋C₄-Alkylrest, eine Nitrogruppe, einen CO₂C₁-C₄-Alkylrest, einen C₁-C₄-Alkoxyrest, einen SC₁-C₄-Alkylrest, CN, einen SO₂C₁-C₄-Alkylrest, SO₂NHR⁷, einen NHSO₂C₁-C₄-Alkylrest oder CₙF₂ₙ₊₁ steht, n für 1 bis 3 steht, p für 0 bis 3 steht und R⁷ ein Wasserstoffatom, ein C₁-C₄-Alkylrest oder (CH₂)₀₋₄-Phenylrest ist; oder
c) Umsetzen einer Verbindung der Formel (II) wie vorstehend definiert mit einer Verbindung der Formel (V): 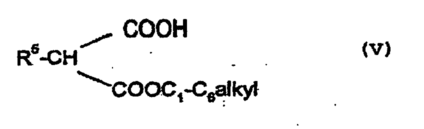 wobei R⁵ wie in Anspruch 1 definiert ist, in Gegenwart einer Base; oder
d) Umsetzen einer Verbindung der Formel (VI): 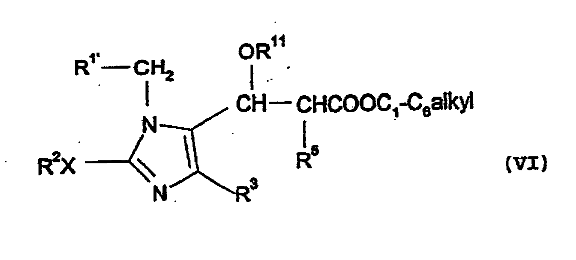 wobei R², R³, R⁵ und X wie in Anspruch 1 definiert sind, R^{1'} für R¹ wie in Anspruch 1 definiert, steht, mit der Ausnahme, dass die Substituenten am Rest R^{1'} keine Tetrazol-5-ylgruppe, kein OH oder CO₂H einschließen und R¹¹ für COCH₃ oder SO₂CH₃ steht, mit einer Base; oder
e) Umsetzen einer Verbindung der Formel (II) wie vorstehend definiert mit einer geeigneten heterocyclischen Essigsäure in Acetanhydrid in Gegenwart einer Base;
und anschließend, wenn nötig
(i) bei Verbindungen der Formel (I), in denen der Rest R¹ mit einer Hydroxygruppe substituiert ist, Entfernen der Schutzgruppen der Verbindungen der Formel (I), in denen der Rest R¹ mit einem C₁-C₄-Alkoxyrest substituiert ist; oder
(ii) bei Verbindungen der Formel (I), in denen der Rest R¹ mit einer Carboxygruppe substituiert ist, Hydrolysieren der Verbindungen der Formel (I), in denen der Rest R¹ mit einem CO₂C₁₋₄-Alkylrest substituiert ist; oder
(iii) bei Verbindungen der Formel (I), in denen der Rest R¹ mit einer Tetrazol-5-ylgruppe substituiert ist, Behandeln der Verbindung der Formel (I), in der der Rest R¹ mit einer Carboxygruppe substituiert ist, mit einem Halogenierungsmittel, anschließend Umwandeln in das primäre Amid durch Umsetzung mit Ammoniak, Dehydrieren mit Oxalylchlorid/Dimethylformamid und Umsetzen mit Azid; oder
(iv) bei Verbindungen der Formel (I), in denen R⁶ für -COOH steht, Hydrolysieren der Verbindungen der Formel (I), in denen R⁶ ein -COOC₁-C₆-Alkylrest ist;
und gegebenenfalls anschließendes Bilden eines pharmazeutisch verträglichen Salzes.

2. Verfahren gemäß Anspruch 1, wobei die Verbindung der Formel (I):
(E)-3-[2-n-Butyl-1-{(4-carboxy-3-chlorphenyl)methyl}-1H-imidazol-5-yl]-2-(2-thienyl)methyl-2-propensäure;
(E)-3-[2-n-Butyl-1-{(2-chlorphenyl)methyl}-1H-imidazol-5-yl]-2-(2-thienyl)methyl-2-propensäure;
(E)-3-[2-n-Butyl-1-{(4-carboxy-2-chlorphenyl)methyl}-1H-imidazol-5-yl]-2-(2-thienyl)methyl-2-propensäure;
(E)-3-[2-n-Butyl-1-{(4-carbomethoxyphenyl)methyl}-1H-imidazol-5-yl]-2-(2-thienyl)methyl-2-propensäure;
(E)-3-[2-n-Hexyl-1-{(4-carboxyphenyl)methyl}-1H-imidazol-5-yl]-2-(2-thienyl)methyl-2-propensäure;
(E)-3-[2-n-Propyl-1-{(4-carboxyphenyl)methyl}-1 H-imidazol-5-yl]-2-(2-thienyl)methyl-2-propensäure;
(E)-3-[2-n-Butyl-1-{(2-nitrophenyl)methyl}-1H-imidazol-5-yl]-2-(2-thienyl)methyl-2-propensäure;
(E)-3-[2-n-Butyl-1-{(2-chlorphenyl)methyl}-1H-imidazol-5-yl]-2-(3-thienyl)methyl-2-propensäure;
(E)-3-[2-n-Butyl-1-{(2-chlorphenyl)methyl}-1H-imidazol-5-yl]-2-(5-methyl-2-thienyl)methyl-2-propensäure;
(E)-3-[2-n-Butyl-1-{(2-cyanophenyl)methyl}-1H-imidazol-5-yl]-2-(2-thienyl)methyl-2-propensäure;
(E)-3-[2-n-Butyl-1-{(4-methoxy-3-methylphenyl)methyl}-1H-imidazol-5-yl]-2-(2-thienyl)methyl-2-propensäure;
(E)-3-[2-n-Butyl-1-{(2-chlorphenyl)methyl}-1H-imidazol-5-yl]-2-(5-methoxy-2-thienyl)methyl-2-propensäure;
(E)-3-[2-n-Butyl-1-{(2,3-dichlorphenyl)methyl}-1H-imidazol-5-yl]-2-(2-thienyl)methyl-2-propensäure; oder
(E)-3-[2-n-Butyl-1-{(2-trifluormethylphenyl)methyl}-1H-imidazol-5-yl]-2-(2-thienyl)methyl-2-propensäure; oder ein pharmazeutisch verträgliches Salz davon ist.

3. Verbindung der Formel (I): 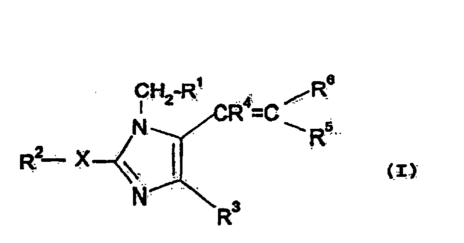 in der
R¹ eine Phenylgruppe ist, welche unsubstituiert oder mit einem bis drei Substituenten substituiert ist, welche ausgewählt sind aus einem Chloratom, Fluoratom, einer Trifluormethyl-, Nitro-, Methyl-, Methoxy-, Hydroxygruppe, einem Sulfamido-, Carboxy-, Carbo-C₁₋₄-alkoxy-, Carbamoyl-, Cyano- oder Tetrazol-5-ylrest;
X eine Einfachbindung ist;
R² für einen C₂-C₈-Alkylrest steht;
R³ ein Wasserstoffatom ist;
R⁴ ein Wasserstoffatom ist;
R⁵ für eine Thienylmethylgruppe, die gegebenenfalls mit einer Methyl- oder Methoxygruppe substituiert ist, steht; und
R⁶ für COOH steht, wobei die Verbindung der Formel (I) das E(trans)-Isomer ist;
oder ein pharmazeutisch verträgliches Salz davon.

4. Verbindung gemäß Anspruch 3 zur Verwendung als Medikament.

5. Arzneimittel, welches eine Verbindung gemäß Anspruch 3 und einen pharmazeutisch verträglichen Träger umfasst.

6. Verwendung einer Verbindung der Formel (I) oder eines pharmazeutisch verträglichen Salzes davon wie in Anspruch 3 definiert, zur Herstellung eines Medikaments zur Behandlung von Erkrankungen, bei denen ein Faktor der Angiotensin II-Rezeptorantagonismus ist.

7. Verwendung einer Verbindung der Formel (I) oder eines pharmazeutisch verträglichen Salzes davon wie in Anspruch 3 definiert, zur Herstellung eines Medikaments zur Behandlung von Hypertonie, dekompensierter Herzinsuffizienz oder Nierenversagen.

## Revendications (Revendications pour l'(les) Etat(s) contractant(s) suivant(s): AT, BE, CH, DE, DK, FR, GB, IT, LI, LU, NL, SE)

1. Composé de formule (I) : 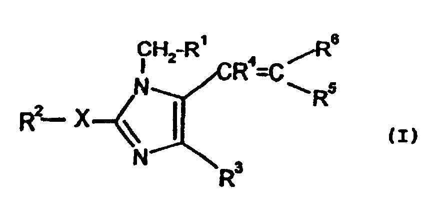 dans laquelle :
R¹ représente un groupe phényle non substitué ou substitué avec un à trois substituants choisis entre des substituants chloro, fluoro, trifluorométhyle, nitro, méthyle, méthoxy, hydroxy, sulfamido, carboxy, carbo(alkoxy en C₁ à C₄), carbamoyle, cyano et tétrazole-5-yle ;
R² représente un groupe alkyle en C₂ à C₈ ;
X représente une liaison simple ;
R³ représente un atome d'hydrogène ;
R⁴ représente un atome d'hydrogène ;
R⁵ représente un groupe thiénylméthyle facultativement substitué avec un substituant méthyle ou méthoxy ; et
R⁶ représente un groupe COOH ;
ou un de ses sels pharmaceutiquement acceptables, le composé de formule (I) étant l'isomère E(trans).

2. Composé suivant la revendication 1, qui est :
l'acide (E)-3-[2-n-butyl-1-{(4-carboxy-3-chlorophényl)méthyl}-1H-imidazole-5-yl]-2-(2-thiényl)méthyl-2-propénoïque ;
l'acide (E)-3-[2-n-butyl-1-{(2-chlorophényl)méthyl}-1H-imidazole-5-yl]-2-(2-thiényl)méthyl-2-propénoïque ;
l'acide (E)-3-[2-n-butyl-1-{(4-carboxy-2-chlorophényl)méthyl}-1H-imidazole-5-yl]-2-(2-thiényl)méthyl-2-propénoique ;
l'acide (E)-3-[2-n-butyl-1-{(4-carbométhoxyphényl)méthyl}-1H-imidazole-5-yl]-2-(2-thiényl)méthyl-2-propénoïque ;
l'acide (E)-3-[2-n-hexyl-1-{(4-carboxyphényl)méthyl}-1H-imidazole-5-yl]-2-(2-thiényl)méthyl-2-propénoïque ;
l'acide (E)-3-[2-n-propyl-1-{(4-carboxyphényl)méthyl}-1H-imidazole-5-yl]-2-(2-thiényl)méthyl-2-propénoïque ;
l'acide (E)-3-[2-n-butyl-1-{(2-nitrophényl)méthyl}-1H-imidazole-5-yl]-2-(2-thiényl)méthyl-2-propénoïque ;
l'acide (E)-3-[2-n-butyl-1-{(2-chlorophényl)méthyl}-1H-imidazole-5-yl]-2-(3-thiényl)méthyl-2-propénoïque ;
l'acide (E)-3-[2-n-butyl-1-{(2-chlorophényl)méthyl}-1H-imidazole-5-yl]-2-(5-méthyl-2-thiényl)méthyl-2-propénoïque ;
l'acide (E)-3-[2-n-butyl-1-{(2-cyanophényl)méthyl}-1H-imidazole-5-yl]-2-(2-thiényl)méthyl-2-propénoïque ;
l'acide (E)-3-[2-n-butyl-1-{(4-méthoxy-3-méthylphényl)méthyl}-1H-imidazole-5-yl]-2-(2-thiényl)méthyl-2-propénoïque ;
l'acide (E)-3-[2-n-butyl-1-{(2-chlorophényl)méthyl}-1H-imidazole-5-yl]-2-(5-méthoxy-2-thiényl)méthyl-2-propénoïque ;
l'acide (E)-3-[2-n-butyl-1-{(2,3-dichlorophényl)méthyl}-1H-imidazole-5-yl]-2-(2-thiényl)méthyl-2-propénoïque ; ou
l'acide (E)-3-[2-n-butyl-1-{(2-trifluorométhylphényl)méthyl}-1H-imidazole-5-yl]-2-(2-thiényl)méthyl-2-propénoïque ; ou un de ses sels pharmaceutiquement acceptables.

3. Composé suivant la revendication 1 ou 2, destiné à être utilisé comme médicament.

4. Composition pharmaceutique qui comprend un composé suivant la revendication 1 ou la revendication 2 et un support pharmaceutiquement acceptable.

5. Procédé pour la préparation d'un composé de formule (I) ou d'un de ses sels pharmaceutiquement acceptables répondant à la définition suivant la revendication 1 ou la revendication 2, procédé qui comprend :
a) la réaction d'un composé de formule (II) : 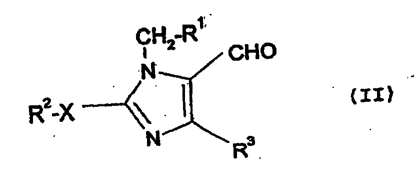 dans laquelle R², R³ et X répondent aux définitions suivant la revendication 1, et R¹ répond à la définition suivant la revendication 1, sauf que les substituants sur le groupe R¹ ne comprennent pas des substituants tétrazole-5-yle, OH ou CO₂H, avec un composé de formule (alkoxy en C₁ à C₄)₂P(O)CH(R⁵)-COO(alkyle en C₁ à C₆), dans lequel R⁵ répond à la définition suivant la revendication 1, en présence d'une base ; ou
b) la réaction d'un composé de formule (III) : 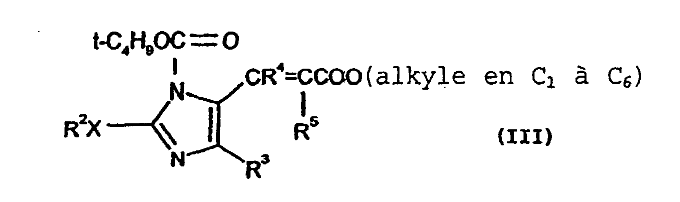 dans laquelle R², R³, R⁴, R⁵ et X répondent aux définitions suivant la revendication 1, avec un composé de formule (IV) : 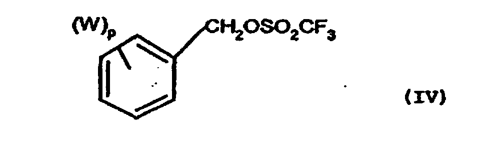 dans laquelle W représente un groupe Cl, Br, F, I, alkyle en C₁ à C₄, nitro, CO₂(alkyle en C₁ à C₄), alkoxy en C₁ à C₄, S(alkyle en C₁ à C₄), CN, SO₂(alkyle en C₁ à C₄), SO₂NHR⁷, NHSO₂(alkyle en C₁ à C₄) ou CₙF₂ₙ₊₁, n a une valeur de 1 à 3, p a une valeur de 0 à 3 et R⁷ représente un atome d'hydrogène, un groupe alkyle en C₁ à C₄ ou (CH₂)₀₋₄phényle ; ou
c) la réaction d'un composé de formule (II) répondant à la définition précitée avec un composé de formule (V) : 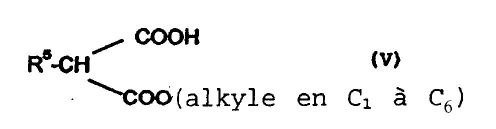 dans laquelle R⁵ répond à la définition suivant la revendication 1, en présence d'une base ; ou
d) la réaction d'un composé de formule (VI) : 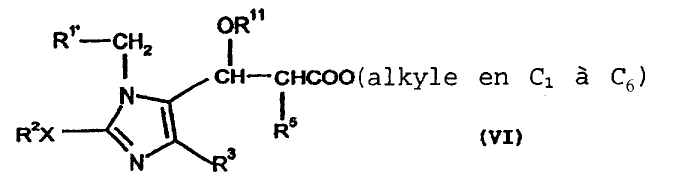 dans laquelle R², R³, R⁵ et X répondent aux définitions suivant la revendication 1, R^{1'} représente un groupe R¹ répondant à la définition suivant la revendication 1, sauf que les substituants sur le groupe R^{1'} ne comprennent pas de substituants tétrazole-5-yle, OH ou CO₂H et R¹¹ représente un groupe COCH₃ ou SO₂CH₃, avec une base ; ou
e) la réaction d'un composé de formule (II) répondant à la définition précitée avec un acide acétique hétérocyclique approprié dans de l'anhydride acétique en présence d'une base ;
et ensuite, lorsque cela est nécessaire :
(i) pour des composés de formule (I) dans lesquels le groupe R¹ est substitué avec un substituant hydroxy, la suppression de la protection des composés de formule (I) dans lesquels le groupe R¹ est substitué avec un groupe alkoxy en C₁ à C₄; ou
(ii) pour des composés de formule (I) dans lesquels le groupe R¹ est substitué avec un substituant carboxy, l'hydrolyse des composés de formule (I) dans lesquels le groupe R¹ est substitué avec un substituant CO₂(alkyle en C₁ à C₄) ; ou
(iii) pour des composés de formule (I) dans lesquels le groupe R¹ est substitué avec un groupe tétrazole-5-yle, le traitement du composé de formule (I) dans lequel le groupe R¹ est substitué avec un substituant carboxy avec un agent d'halogénation, puis la conversion en l'amide primaire dans une réaction avec l'ammoniac, une déshydratation avec du chlorure d'oxalyle/diméthylformamide et une réaction avec un azoture ; ou
(iv) pour des composés de formule (I) dans lesquels R⁶ représente un groupe -COOH, l'hydrolyse des composés de formule (I) dans lesquels R⁶ représente un groupe -COO(alkyle en C₁ à C₆) ;
et ensuite, facultativement, la formation d'un sel pharmaceutiquement acceptable.

6. Utilisation d'un composé de formule (I) ou d'un de ses sels pharmaceutiquement acceptables répondant à la définition suivant la revendication 1 ou la revendication 2 dans la production d'un médicament destiné au traitement de maladies dans lesquelles l'antagonisme des récepteurs d'angiotensine II est un facteur.

7. Utilisation d'un composé de formule (I) ou d'un de ses sels pharmaceutiquement acceptables répondant à la définition suivant la revendication 1 ou la revendication 2 dans la production d'un médicament destiné au traitement de l'hypertension, de l'insuffisance cardiaque congestive ou de l'insuffisance rénale.

## Revendications (Revendications pour l'(les) Etat(s) contractant(s) suivant(s): ES, GR)

1. Procédé pour la préparation d'un composé de formule (I) : 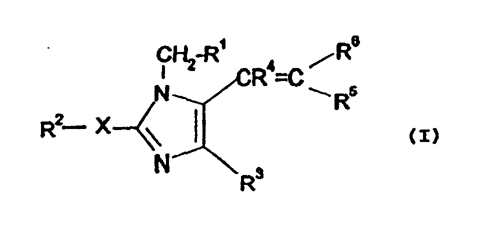 dans laquelle :
R¹ représente un groupe phényle non substitué ou substitué avec un à trois substituants choisis entre des substituants chloro, fluoro, trifluorométhyle, nitro, méthyle, méthoxy, hydroxy, sulfamido, carboxy, carbo(alkoxy en C₁ à C₄), carbamoyle, cyano et tétrazole-5-yle ;
X représente une liaison simple ;
R² représente un groupe alkyle en C₂ à C₈ ;
R³ représente un atome d'hydrogène ;
R⁴ représente un atome d'hydrogène ;
R⁵ représente un groupe thiénylméthyle facultativement substitué avec un substituant méthyle ou méthoxy ; et
R⁶ représente un groupe COOH, le composé de formule (I) étant l'isomère E(trans), ou un de ses sels pharmaceutiquement acceptables ;
procédé qui comprend :
a) la réaction d'un composé de formule (II) : 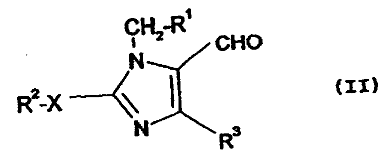 dans laquelle R², R³ et X répondent aux définitions suivant la revendication 1, et R¹ répond à la définition suivant la revendication 1, sauf que les substituants sur le groupe R¹ ne comprennent pas des substituants tétrazole-5-yle, OH ou CO₂H, avec un composé de formule (alkoxy en C₁ à C₄)₂P(O)CH(R⁵)-COO(alkyle en C₁ à C₆), dans lequel R⁵ répond à la définition suivant la revendication 1, en présence d'une base ; ou
b) la réaction d'un composé de formule (III) : 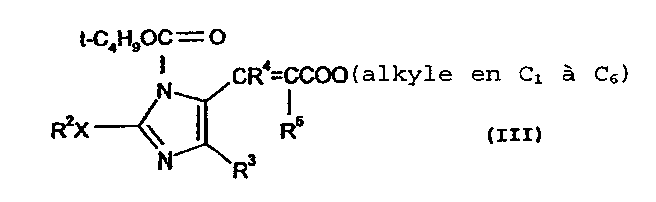 dans laquelle R², R³, R⁴, R⁵ et X répondent aux définitions suivant la revendication 1, avec un composé de formule (IV) : 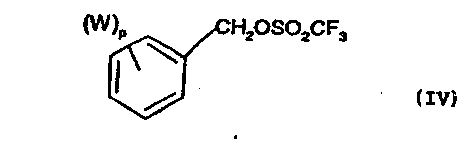 dans laquelle W représente un groupe Cl, Br, F, I, alkyle en C₁ à C₄, nitro, CO₂(alkyle en C₁ à C₄), alkoxy en C₁ à C₄, S(alkyle en C₁ à C₄), CN, SO₂(alkyle en C₁ à C₄), SO₂NHR⁷, NHSO₂(alkyle en C₁ à C₄) ou CₙF₂ₙ₊₁, n a une valeur de 1 à 3, p a une valeur de 0 à 3 et R⁷ représente un atome d'hydrogène, un groupe alkyle en C₁ à C₄ ou (CH₂)₀₋₄phényle ; ou
c) la réaction d'un composé de formule (II) répondant à la définition précitée avec un composé de formule (V) : 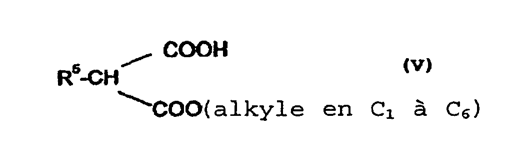 dans laquelle R⁵ répond à la définition suivant la revendication 1, en présence d'une base ; ou
d) la réaction d'un composé de formule (VI) : 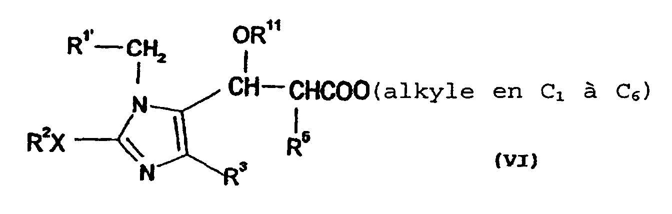 dans laquelle R², R³, R⁵ et X répondent aux définitions suivant la revendication 1, R^{1'} représente un groupe R¹ répondant à la définition suivant la revendication 1, sauf que les substituants sur le groupe R^{1'} ne comprennent pas de substituants tétrazole-5-yle, OH ou CO₂H et R¹¹ représente un groupe COCH₃ ou SO₂CH₃, avec une base ; ou
e) la réaction d'un composé de formule (II) répondant à la définition précitée avec un acide acétique hétérocyclique approprié dans de l'anhydride acétique en présence d'une base ;
et ensuite, si nécessaire :
(i) pour des composés de formule (I) dans lesquels le groupe R¹ est substitué avec un substituant hydroxy, la suppression de la protection des composés de formule (I) dans lesquels le groupe R¹ est substitué avec un groupe alkoxy en C₁ à C₄ ; ou
(ii) pour des composés de formule (I) dans lesquels le groupe R¹ est substitué avec un substituant carboxy, l'hydrolyse des composés de formule (I) dans lesquels le groupe R¹ est substitué avec un substituant CO₂(alkyle en C₁ à C₄) ; ou
(iii) pour des composés de formule (I) dans lesquels le groupe R¹ est substitué avec un groupe tétrazole-5-yle, le traitement du composé de formule (I) dans lequel le groupe R¹ est substitué avec un substituant carboxy avec un agent d'halogénation, avec ensuite la conversion à l'amide primaire dans une réaction avec l'ammoniac, une déshydratation avec du chlorure d'oxalyle/diméthylformamide et une réaction avec un azoture ; ou
(iv) pour des composés de formule (I) dans lesquels R⁶ représente un groupe -COOH, l'hydrolyse des composés de formule (I) dans lesquels R⁶ représente un groupe -COO(alkyle en C₁ à C₆) ;
et ensuite, facultativement, la formation d'un sel pharmaceutiquement acceptable.

2. Procédé suivant la revendication 1, dans lequel le composé de formule (I) est :
l'acide (E)-3-[2-n-butyl-1-{(4-carboxy-3-chlorophényl)méthyl}-1H-imidazole-5-yl]-2-(2-thiényl)méthyl-2-propénoïque ;
l'acide (E)-3-[2-n-butyl-1-{(2-chlorophényl)méthyl}-1H-imidazole-5-yl]-2-(2-thiényl)méthyl-2-propénoïque ;
l'acide (E)-3-[2-n-butyl-1-{(4-carboxy-2-chlorophényl)méthyl}-1H-imidazole-5-yl]-2-(2-thiényl)méthyl-2-propénoïque ;
l'acide (E)-3-[2-n-butyl-1-{(4-carbométhoxyphényl)méthyl}-1H-imidazole-5-yl]-2-(2-thiényl)méthyl-2-propénoïque ;
l'acide (E)-3-[2-n-hexyl-1-{(4-carboxyphényl)méthyl}-1H-imidazole-5-yl]-2-(2-thiényl)méthyl-2-propénoïque ;
l'acide (E)-3-[2-n-propyl-1-{(4-carboxyphényl)méthyl}-1H-imidazole-5-yl]-2-(2-thiényl)méthyl-2-propénoique ;
l'acide (E)-3-[2-n-butyl-1-{(2-nitrophényl)méthyl}-1H-imidazole-5-yl]-2-(2-thiényl)méthyl-2-propénoïque ;
l'acide (E)-3-[2-n-butyl-1-{(2-chlorophényl)méthyl}-1H-imidazole-5-yl]-2-(3-thiényl)méthyl-2-propénoïque ;
l'acide (E)-3-[2-n-butyl-1-{(2-chlorophényl)méthyl}-1H-imidazole-5-yl]-2-(5-méthyl-2-thiényl)méthyl-2-propénoïque ;
l'acide (E)-3-[2-n-butyl-1-{(2-cyanophényl)méthyl}-1H-imidazole-5-yl]-2-(2-thiényl)méthyl-2-propénoïque ;
l'acide (E)-3-[2-n-butyl-1-{(4-méthoxy-3-méthylphényl)méthyl}-1H-imidazole-5-yl]-2-(2-thiényl)méthyl-2-propénoïque ;
l'acide (E)-3-[2-n-butyl-1-{(2-chlorophényl)méthyl}-1H-imidazole-5-yl]-2-(5-méthoxy-2-thiényl)méthyl-2-propénoïque ;
l'acide (E)-3-[2-n-butyl-1-{(2,3-dichlorophényl)méthyl}-1H-imidazole-5-yl]-2-(2-thiényl)méthyl-2-propénoïque ; ou
l'acide (E)-3-[2-n-butyl-1-{(2-trifluorométhylphényl)méthyl}-1H-imidazole-5-yl]-2-(2-thiényl)méthyl-2-propénoïque ; ou un de ses sels pharmaceutiquement acceptables.

3. Composé de formule (I) : 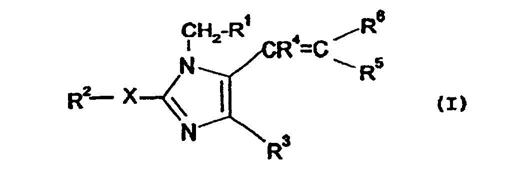 dans laquelle :
R¹ représente un groupe phényle non substitué ou substitué avec un à trois substituants choisis entre des substituants chloro, fluoro, trifluorométhyle, nitro, méthyle, méthoxy, hydroxy, sulfamido, carboxy, carbo(alkoxy en C₁ à C₄), carbamoyle, cyano et tétrazole-5-yle ;
X représente une liaison simple ;
R² représente un groupe alkyle en C₂ à C₈ ;
R³ représente un atome d'hydrogène ;
R⁴ représente un atome d'hydrogène ;
R⁵ représente un groupe thiénylméthyle facultativement substitué avec un substituant méthyle ou méthoxy ; et
R⁶ représente un groupe COOH, le composé de formule (I) étant l'isomère E(trans), ou un de ses sels pharmaceutiquement acceptables.

4. Composé suivant la revendication 3, destiné à être utilisé comme médicament.

5. Composition pharmaceutique qui comprend un composé suivant la revendication 3 et un support pharmaceutiquement acceptable.

6. Utilisation d'un composé de formule (I) ou d'un de ses sels pharmaceutiquement acceptables répondant à la définition suivant la revendication 3 dans la production d'un médicament destiné au traitement de maladies dans lesquelles l'antagonisme des récepteurs d'angiotensine II est un facteur.

7. Utilisation d'un composé de formule (I) ou d'un de ses sels pharmaceutiquement acceptables répondant à la définition suivant la revendication 3 dans la production d'un médicament destiné au traitement de l'hypertension, de l'insuffisance cardiaque congestive ou de l'insuffisance rénale.
